# FASCICULE DE BREVET EUROPEEN

(11) **EP 2 691 402 B1**
(45) Date de publication et mention de la délivrance du brevet: **08.05.2019**
(21) Numéro de dépôt: 12711632.5
(22) Date de dépôt: 28.03.2012
(51) Int. Cl.: C07F 9/38, A61K 31/662, A61P 35/00

(54) **DERIVES D'ACIDE HYDROXYBISPHOSPHONIQUE BIFONCTIONNELS**
BIFUNKTIONELLE HYDROXY-BISPHOSPHONSÄUREDERIVATE
BIFUNCTIONAL HYDROXY-BISPHOSPHONIC ACID DERIVATIVES

(30) Priorité: 28.03.2011 FR 1152546
(43) Date de publication de la demande: 05.02.2014
(73) Titulaire: Atlanthera, 44800 Saint Herblain (FR)
(72) Inventeur: EGOROV, Maxim, 44340 Bouguenais (FR); GOUJON, Jean-Yves, 44590 Derval (FR); LE BOT, Ronan, 44100 Nantes (FR)
(74) Mandataire: Regimbeau
(86) Numéro de dépôt international: PCT/EP2012/055569
(87) Numéro de publication internationale: WO 2012/130911

(56) Documents cités:
- EP-A1- 0 387 194
- WO-A1-2009/083614
- FR-A1- 2 926 080

## Description

La présente invention concerne des dérivés d'acide hydroxy-bisphosphonique bifonctionnels, leur procédé de synthèse, les compositions pharmaceutiques les contenant, et leur utilisation en tant que médicament, ainsi que la vectorisation de molécules d'intérêt thérapeutique ou diagnostique par un vecteur ciblant le tissu osseux.

Le tissu osseux est un tissu conjonctif composé d'une fraction minérale constituée de phosphate de calcium sous forme de cristaux d'hydroxyapatite (Ca₁₀(PO₄)₆(OH)₂) et d'une fraction organique contenant une matrice extracellulaire et des cellules spécialisées.

Le tissu osseux est en perpétuel remaniement grâce à un processus appelé remodelage osseux. Il se caractérise par une phase d'apposition due à l'activité des ostéoblastes qui synthétisent une nouvelle matrice organique et induisent sa minéralisation (Owen et al., Curr. Opin. Nephrol. Hypertens. 1998, 7, 363) et une phase de dégradation assurée par les ostéoclastes qui résorbent la matrice organique et dissolvent le minéral (Roodman et al., Endocr. Rev. 1996, 17, 308). Ce processus physiologique permet de maintenir l'homéostasie phosphocalcique et la masse osseuse (Manologas et al., Endocriv. Rev. 2000, 21, 115) et de s'adapter aux contraintes mécaniques. Tout dérèglement de cet équilibre est lié à l'apparition de pathologies ostéocondensantes, telles que l'ostéopétrose, ou, le plus souvent, ostéolytiques pouvant être d'origine tumorale (avec des tumeurs primaires, comme l'ostéosarcome, ou secondaires, comme les métastases osseuses) ou non dans le cas de pathologies métaboliques comme l'ostéoporose.

Les bisphosphonates (forme basique de dérivés d'acide bisphosphonique dont font partie les dérivés d'acide hydroxy-bisphosphonique) sont des analogues synthétiques des pyrophosphates endogènes pour lesquels la chaîne P-O-P a été remplacée par une chaîne P-C-P, conduisant à des composés métaboliquement stables qui représentent un outil thérapeutique efficace des ostéolyses (Heymann et al., Trends Mol. Med., 2004, 10, 337).

Ces molécules ont tout d'abord été utilisées pour leur capacité à cibler le tissu osseux. De la même manière que les pyrophosphates, les bisphosphonates ont une forte affinité pour la partie minérale de l'os (affinité entre les groupements phosphates et le calcium de la partie minérale de l'os) et peuvent moduler à forte dose le processus de calcification. L'intérêt de telles substances a été mis en évidence pour le traitement de divers dysfonctionnements du métabolisme osseux. Les bisphosphonates sont utilisés en particulier pour traiter les pathologies impliquant une résorption osseuse excessive conduisant d'une part à une hypercalcémie et d'autre part à des atteintes osseuses à l'origine de douleurs et de fractures.

Ainsi, leur utilisation s'est imposée depuis une dizaine d'années pour le traitement de l'ostéoporose, de l'hypercalcémie d'origine tumorale ou non, ainsi que pour des pathologies ostéolytiques tumorales telles que le myélome multiple ou les métastases osseuses secondaires d'un carcinome prostatique ou mammaire.

Les études de structure-activité, développées à ce jour, ont clairement montré que la capacité des bisphosphonates à inhiber la résorption osseuse dépendait de deux facteurs structuraux :
- les groupes phosphonates (et hydroxyle dans le cas d'hydroxy-bisphosphonates), essentiels pour une bonne affinité du composé avec la partie minérale de l'os,
- la chaîne latérale RES, spécifique d'une cible moléculaire, qui détermine l'activité biologique associée à la molécule.

Dérivé d'hydroxy-bisphosphonate :

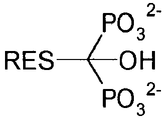

La demande de brevet FR 2 926 081 décrit notamment des dérivés d'acide hydroxy-bisphosphonique bifonctionnels comprenant un principe actif lié à une fonction acide hydroxybisphosphonique par l'intermédiaire d'un bras espaceur, permettant ainsi de cibler le tissu osseux. Cependant, ces dérivés sont préparés à partir du principe actif lui-même par construction progressive du bras espaceur. Ce procédé n'est donc pas économique et entraîne la perte de grandes quantités du principe actif, produit ayant toujours un coût élevé.

Les inventeurs ont ainsi développé de nouveaux dérivés d'acide hydroxy-bisphosphonique bifonctionnels pouvant être synthétisés par simple greffage du bras espaceur comprenant la fonction hydroxybisphosphonique sur le principe actif éventuellement fonctionnalisé. Dans le cadre de la présente invention, le principe actif, éventuellement fonctionnalisé, est lié au bras espaceur par l'intermédiaire d'une fonction imine, permettant ainsi de préparer les dérivés d'acide hydroxy-bisphosphonique bifonctionnels de manière plus simple et économique tout en préservant l'activité thérapeutique ou diagnostique de ces dérivés.

La présente description divulgue des dérivés d'acide hydroxy-bisphosphonique bifonctionnels répondant à la formule générale (I) suivante : 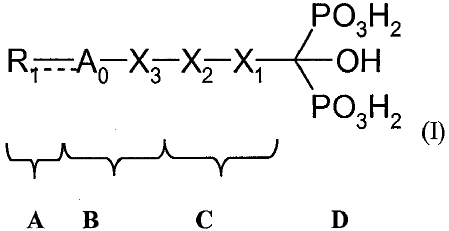 pour laquelle :
- -̅-̅-̅-̅-A₀ représente une liaison simple, A₀ étant alors absent, ou un groupe =N ou =CR₀, A₀ représentant alors N ou CR₀, avec R₀ = H ou (C₁-C₆)-alkyle,
- R₁ représente un résidu d'une molécule d'intérêt thérapeutique ou diagnostique,
- X₁ représente une chaîne -X₄-A₁-(CH₂)ₙ-A₂-(CH₂)ₘ- (X₄ étant lié à X₂),
- X₂ représente une fonction imine (-C=N- ou -N=C-),
- X₃ représente une liaison simple ou une chaîne (C₁-C₂₀)-alkyle, notamment (C₁-C₁₅)-alkyle, en particulier (C₁-C₁₀)-alkyle, et de préférence (C₁-C₄)-alkyle, éventuellement interrompue et/ou suivie et/ou remplacée par un ou plusieurs motifs choisis dans le groupe constitué par les groupes aryle, hétéroaryle, cycloalkyle, hétérocyclique, -C=C-, -C(R₇)=C(R₈)-, -O-, -S-, -NR₉-, -C(O)-, -C(S)-, -C=N-, -N=C-, -C=C-, -C≡C-, -OC(O)-, -C(O)O-, -SC(O)-, -C(O)S-, -N(R₁₀)C(O)- et -C(O)N(R₁₁)-, les noyaux aryle, hétéroaryle et hétérocyclique étant éventuellement substitués,
- X₄ représente une liaison simple ou un groupe aryle ou hétéroaryle éventuellement substitué,
- A₁ représente une liaison simple, O, S, NR₂₇, -C(O)-, -C(S)-, -C=N-, -N=C-, -C=C-, -C≡C-, -OC(O)-, -C(O)O-, -SC(O)-, -C(O)S-, -N(R₂₈)C(O)- ou -C(O)N(R₂₉)-,
- A₂ représente une liaison simple, O, S, NR₃₀, -C(O)-, -C(S)-, -C=N-, -N=C-, -C=C-, -C≡C-, -OC(O)-, -C(O)O-, -SC(O)-, -C(O)S-, -N(R₃₁)C(O)-,-C(O)N(R₃₂)-, -O-(CH₂CH₂O)ₖ- ou un groupe -A₉-(CH₂)ₐ-A₁₀-(CH₂)_{b}-A₁₁-,
- A₉ et A₁₁ représentent chacun, indépendamment l'un de l'autre, O, S, ou NR₃₈,
- A₁₀ représente un groupe aryle, hétéroaryle ou hétérocyclique,
- n représente un nombre entier compris entre 0 et 5, notamment compris entre 1 et 5, et notamment 3,
- m représente un nombre entier compris entre 0 et 5, notamment compris entre 1 et 5, et notamment 2,
- k représente un nombre entier compris entre 1 et 10, notamment compris entre 1 et 5,
- a et b représentent, indépendamment l'un de l'autre, un nombre entier compris entre 0 et 5,
- R₇ et R₈ représentent, indépendamment les uns des autres, un atome d'hydrogène ou un groupe (C₁-C₆)alkyle,
- R₉ à R₁₁ représentent indépendamment les uns des autres, un atome d'hydrogène ou un groupe (C₁-C₆)alkyle, cycloalkyle, hétérocyclique, aryle, hétéroaryle ou acyle, et de préférence un atome d'hydrogène ou un groupe (C₁-C₆)alkyle ou aryle, et encore de préférence un atome d'hydrogène ou un groupe (C₁-C₆)alkyle,
- R₂₇ à R₂₉ représentent, indépendamment les uns des autres, un atome d'hydrogène ou un groupe (C₁-C₆)-alkyle, et
- R₃₀ à R₃₂ et R₃₈ représentent, indépendamment les uns des autres, un atome d'hydrogène ou un groupe (C₁-C₆)-alkyle, cycloalkyle, hétérocyclique, aryle, hétéroaryle ou acyle, et de préférence un atome d'hydrogène ou un groupe (C₁-C₆)-alkyle ou aryle, et encore de préférence un atome d'hydrogène ou un groupe (Ci-C₆)-alkyle tel que méthyle,
ou des sels pharmaceutiquement acceptables de ceux-ci.

Les molécules de la présente description sont donc composées de quatre parties distinctes :
▪ une partie D correspondant à la fonction acide hydroxy-bisphosphonique qui va permettre à la molécule de cibler le tissu osseux de part la forte affinité de cette fonction pour la partie minérale de l'os,
▪ une partie C qui est un bras espaceur, sur lequel pourra être greffé le résidu R₁,
▪ une partie B qui est un linker permettant de greffer le résidu R₁ sur le bras espaceur, et enfin
▪ une partie A correspondant au résidu R₁ d'une molécule à activité thérapeutique ou diagnostique, permettant notamment un traitement ciblé d'une pathologie du tissu osseux ou encore un diagnostic, notamment par imagerie de ce tissu osseux.

Par « résidu d'une molécule d'intérêt thérapeutique ou diagnostique », on entend, au sens de la présente description, le résidu obtenu lorsque la molécule d'intérêt thérapeutique ou diagnostique est liée au reste de la molécule, et en particulier au bras espaceur B. Ainsi, la molécule d'intérêt thérapeutique ou diagnostique doit comprendre ou doit être modifiée de manière à comprendre un groupement fonctionnel permettant un couplage avec le bras espaceur, tel qu'un groupement -OH, -SH, -NH, -NH₂, C=O, -CHO, -COOH ou -CONH₂. Il est à noter que, quand -̅-̅-̅-A₀ représente un groupe =N, le résidu comportera avantageusement une fonction C=O ou -CHO qui pourra réagir avec une fonction amine du linker pour former le groupe =N. Lorsque -̅-̅-̅-A₀ représente un groupe =CR₀, la double liaison sera avantageusement liée à un atome d'azote présent sur R₁ pour donner une fonction imine. Ainsi, le résidu comportera avantageusement une fonction amine qui pourra réagir avec une fonction C=O ou -CHO du linker pour former le groupe =CR₀.

Par groupement « alkyle », on entend, au sens de la présente description, une chaîne hydrocarbonée saturée, linéaire ou ramifiée.

Par groupement « (C₁-C₆)alkyle », on entend, au sens de la présente description, un groupement alkyle tel que défini ci-dessus, comportant de 1 à 6 atomes de carbone, comme par exemple un groupement méthyle, éthyle, isopropyle, *tertio*-butyle, pentyle, etc.

Par groupement « (C₁-C₂₀)alkyle, notamment (C₁-C₁₅)-alkyle, en particulier (C₁-C₁₀)-alkyle, et de préférence (C₁-C₄)-alkyle », on entend, au sens de la présente description, un groupement alkyle tel que défini ci-dessus, comportant respectivement de 1 à 20, notamment 1 à 15, en particulier 1 à 10, et de préférence 1 à 4, atomes de carbone, comme par exemple un groupement méthyle, éthyle, isopropyle, *tertio*-butyle, pentyle, hexyle, décyle, etc.

Par « aryle », on entend, au sens de la présente description, un groupement aromatique, comportant notamment de 6 à 20, de préférence de 6 à 10, atomes de carbone, et comprenant un ou plusieurs cycles accolés, comme par exemple un groupement phényle ou naphtyle. Avantageusement, il s'agit du phényle.

Par « hétéroaryle », on entend, au sens de la présente description, un groupe aromatique comprenant un ou plusieurs cycles accolés et comprenant 5 à 10 atomes cycliques dont un ou plusieurs hétéroatomes, avantageusement 1 à 4 et encore plus avantageusement 1 ou 2, tels que par exemple des atomes de soufre, azote, oxygène phosphore ou sélénium et de préférence de soufre, azote ou oxygène, les autres atomes cycliques étant des atomes de carbone. Des exemples de groupes hétéroaryle sont les groupes furyle, thiényle, pyrrolyle, pyridinyle, pyrimidinyle, pyrazolyle, imidazolyle, triazolyle, tétrazolyle indyle ou encore sélénophényle.

Par groupement « cycloalkyle », on entend, au sens de la présente description, une chaîne hydrocarbonée saturée mono- ou poly-cyclique (et notamment bicyclique ou tricyclique) comportant 3 à 10 atomes de carbone cycliques. Lorsqu'il s'agit d'un groupe polycyclique, les cycles peuvent être, deux à deux, accolés, pontés ou joints par une jonction de cycle de type spiro. A titre d'exemple, on peut citer les groupes cyclopropyle, cyclopentyle, cyclohexyle et cycloheptyle.

Par groupement « hétérocyclique », on entend, au sens de la présente description, un cycle à 5 à 10 chaînons, saturé ou non, mais non aromatique, et contenant un ou plusieurs, avantageusement 1 à 4, encore plus avantageusement 1 ou 2, hétéroatomes, tels que par exemple des atomes de soufre, azote ou oxygène. Il peut s'agir notamment du groupe pyrrolidinyle, pipéridinyle, pipérazinyle ou morpholinyle.

Les groupes aryle, hétéroaryle et hétérocyclique, lorsqu'ils sont substitués, peuvent l'être avec un ou plusieurs groupements choisis dans le groupe constitué par un atome d'halogène, NO₂, -CN, -OH, -SH, -NR₁₂R₁₃, -B(OH)₂, -SO₃R₁₄, -COOR₁₅, -C(O)ONR₁₆R₁₇, -OPH(O)OR₁₈, -PH(O)OR₁₉, -OP(O)(OR₂₀)(OR₂₁), -P(O)(OR₂₂)(OR₂₃), -C(O)R₂₄, -PR₂₅R₂₆, (C₁-C₆)alkyle et (C₁-C₆)alcoxy, avec R₁₂ à R₂₄ représentant, indépendamment les uns des autres, un atome d'hydrogène ou un groupe (C₁-C₆)alkyle et R₂₅ et R₂₆ représentant, indépendamment l'un de l'autre, un groupe (C₁-C₆)alkyle.

Par « atome d'halogène », on entend, au sens de la présente description, les atomes de fluor, de chlore, de brome et d'iode.

Par groupement « (C₁-C₆)alcoxy », on entend, au sens de la présente description, un groupe (C₁-C₆)alkyle, tel que défini ci-dessus, lié au reste de la molécule par l'intermédiaire d'un atome d'oxygène. A titre d'exemple, on peut citer les groupes méthoxy, éthoxy, propoxy, isopropoxy, butoxy ou encore tert-butoxy, et en particulier méthoxy.

Par groupement « acyle », on entend désigner, au sens de la présente description, un groupe (C₁-C₆)alkyle, cycloalkyle, hétérocyclique, aryle ou hétéroaryle, et de préférence un groupe (C₁-C₆)alkyle ou aryle, et encore de préférence un groupe (C₁-C₆)alkyle.

Dans la présente invention, on entend désigner par « pharmaceutiquement acceptable » ce qui est utile dans la préparation d'une composition pharmaceutique qui est généralement sûre, non toxique et ni biologiquement ni autrement non souhaitable et qui est acceptable pour une utilisation vétérinaire de même que pharmaceutique humaine.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(3) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique pharmaceutiquement acceptable. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

De préférence, les composés selon l'invention seront sous la forme de sels d'addition de base pharmaceutiquement acceptable telle que NaOH ou KOH, et notamment NaOH.

La présente description divulgue aussi des composés de formule (I) ci-dessus pour laquelle :
- -̅-̅-̅-̅-A₀ représente une liaison simple ou un groupe =N,
- R₁ représente un résidu d'une molécule d'intérêt thérapeutique ou diagnostique,
- X₁ représente une chaîne -X₄-A₁-(CH₂)ₙ-A₂-(CH₂)ₘ-,
- X₂ représente une fonction imine (-C=N- ou -N=C-),
- X₃ représente une liaison simple ou une chaîne (C₁-C₂₀)-alkyle, notamment (C₁-C₁₅)-alkyle, en particulier (C₁-C₁₀)-alkyle, et de préférence (C₁-C₄)-alkyle, éventuellement interrompue et/ou suivie par un ou plusieurs motifs choisis dans le groupe constitué par les groupes aryle, hétéroaryle, cycloalkyle, hétérocyclique, -C≡C-, -C(R₇)=C(R₈)-, -O-, -S-, -NR₉-, -C(O)-, -OC(O)-, -C(O)O-, -SC(O)-, -C(O)S-, -N(R₁₀)C(O)- et -C(O)N(R₁₁)-, les noyaux aryle, hétéroaryle et hétérocyclique étant éventuellement substitués,
- X₄ représente un groupe aryle ou hétéroaryle éventuellement substitué,
- A₁ représente une liaison simple, O, S, NR₂₇, -C(O)-, -OC(O)-, -C(O)O-, -SC(O)-, -C(O)S-, -N(R₂₈)C(O)- ou -C(O)N(R₂₉)-,
- A₂ représente O, S, NR₃₀, -C(O)-, -OC(O)-, -C(O)O-, -SC(O)-, -C(O)S-, -N(R₃₁)C(O)- ou -C(O)N(R₃₂)-,
- n représente un nombre entier compris entre 1 et 5, et notamment 3,
- m représente un nombre entier compris entre 0 et 5, notamment compris entre 1 et 5, et notamment 2,
- R₇ et R₈ représentent, indépendamment les uns des autres, un atome d'hydrogène ou un groupe (C₁-C₆)alkyle,
- R₉ à R₁₁ représentent, indépendamment les uns des autres, un atome d'hydrogène ou un groupe (C₁-C₆)alkyle, cycloalkyle, hétérocyclique, aryle, hétéroaryle ou acyle, et de préférence un atome d'hydrogène ou un groupe (C₁-C₆)alkyle ou aryle, et encore de préférence un atome d'hydrogène ou un groupe (C₁-C₆)alkyle,
- R₂₇ à R₂₉ représentent, indépendamment les uns des autres, un atome d'hydrogène ou un groupe (C₁-C₆)alkyle, et
- R₃₀ à R₃₂ représentent, indépendamment les uns des autres, un atome d'hydrogène ou un groupe (C₁-C₆)alkyle, cycloalkyle, hétérocyclique, aryle, hétéroaryle ou acyle, et de préférence un atome d'hydrogène ou un groupe (C₁-C₆)alkyle ou aryle, et encore de préférence un atome d'hydrogène ou un groupe (C₁-C₆)alkyle tel que méthyle,
ou des sels pharmaceutiquement acceptables de ceux-ci.

La présente description divulgue également des composés de formule (I) dans laquelle, -̅-̅-̅-A₀ représente une liaison simple, A₀ étant alors absent, ou un groupe =N, A₀ représentant alors N.

La présente description divulgue également des composés de formule (I) dans laquelle X₁ ne représente pas une liaison simple.

La présente description divulgue également des composés de formule (I) dans laquelle X₄ représente un groupe aryle ou hétéroaryle éventuellement substitué, et notamment un groupe aryle, tel que phényle, éventuellement substitué.

La présente description divulgue également des composés de formule (I) dans laquelle X₄ représente un groupe phényle, naphtyle ou indolyle éventuellement substitué, le groupe indolyle étant de préférence lié à A₁ par l'intermédiaire de son atome d'azote, et de préférence représente un groupe phényle éventuellement substitué. Les groupes aryle et hétéroaryle, tels que phényle, naphtyle et indolyle, de X₄ pourront être substitués comme défini précédemment, c'est-à-dire avec un ou plusieurs groupements choisis dans le groupe constitué par un atome d'halogène, NO₂, -CN, -OH, -SH, -NR₁₂R₁₃, -B(OH)₂, -SO₃R₁₄, -COOR₁₅, -C(O)ONR₁₆R₁₇, -OPH(O)OR₁₈, -PH(O)OR₁₉, -OP(O)(OR₂₀)(OR₂₁), -P(O)(OR₂₂)(OR₂₃), -C(O)R₂₄, -PR₂₅R₂₆, (C₁-C₆)alkyle et (C₁-C₆)alcoxy, avec R₁₂ à R₂₄ représentant, indépendamment les uns des autres, un atome d'hydrogène ou un groupe (C₁-C₆)alkyle et R₂₅ et R₂₆ représentant, indépendamment l'un de l'autre, un groupe (C₁-C₆)alkyle. Avantageusement, ils seront substitués par un ou plusieurs groupements choisis dans le groupe constitué par un atome d'halogène, NO₂ et (C₁-C₆)alcoxy, et notamment par NO₂.

La présente description divulgue également des composés de formule (I) dans laquelle A₁ représente une liaison simple, O, S, NR₂₇, -C(O)-, -OC(O)-, -C(O)O-, -SC(O)-, -C(O)S-, -N(R₂₈)C(O)- ou -C(O)N(R₂₉)-.

La présente description divulgue également des composés de formule (I) dans laquelle A₁ représente une liaison simple, O, S ou NR₂₇, notamment une liaison simple ou O, et de préférence O.

La présente description divulgue également des composés de formule (I) dans laquelle A₁ représente une liaison simple quand X₄ représente un groupe hétéroaryle comprenant un atome d'azote, tel qu'un groupe indolyle, et lié à A₁ par l'intermédiaire de cet atome d'azote. A₁ sera de préférence différent d'une liaison simple, et notamment représentera O, S ou NR₂₇, et en particulier un atome d'oxygène, quand X₄ représentera un aryle, tel qu'un groupe phényle ou naphtyle, ou un hétéroaryle lié à A₁ par l'intermédiaire d'un atome de carbone.

La présente description divulgue également des composés de formule (I) dans laquelle A₂ représente O, S, NR₃₀, -C(O)-, -OC(O)-, -C(O)O-, -SC(O)-, -C(O)S-, -N(R₃₁)C(O)- ou -C(O)N(R₃₂)-; ou encore représente -O-(CH₂CH₂O)ₖ-.

La présente description divulgue également des composés de formule (I) dans laquelle A₂ représente O, S ou NR₃₀, et de préférence NR₃₀, avec R₃₀ tel que défini précédemment et représentant notamment un atome d'hydrogène ou un groupe (C₁-C₆)-alkyle ou aryle, et de préférence un atome d'hydrogène ou un groupe (C₁-C₆)-alkyle tel que méthyle. A₂ pourra représenter également un groupe -O-(CH₂CH₂O)ₖ- avec k tel que défini précédemment et notamment représentant 2.

La présente description divulgue également des composés de formule (I) dans laquelle A₂ représente un groupe NR₃₀ avec R₃₀ représentant, un atome d'hydrogène ou un groupe (C₁-C₆)-alkyle, et de préférence un groupe (C₁-C₆)-alkyle tel que méthyle.

La présente description divulgue également des composés de formule (I) dans laquelle X₁ représente une chaîne de formule -X₄-A₁-(CH₂)ₙ-A₂-(CH₂)ₘ- avec :
- X₄ représentant un groupe phényle, éventuellement substitué comme défini précédemment et notamment par NO₂,
- A₁ représentant un atome d'oxygène,
- A₂ représentant un groupe NR₃₀ avec R₃₀ représentant un groupe (C₁-C₆)-alkyle tel que méthyle,
- n représentant 3, et
- m représentant 2.

La présente description divulgue également des composés de formule (I) dans laquelle X₂ représente une fonction -N=CH-.

La présente description divulgue également des composés de formule (I) dans laquelle -̅-̅-̅-A₀ représente une liaison simple.

La présente description divulgue également des composés de formule (I) dans laquelle X₃ représenterun groupe -A₃-, -A₃-A₄-A₅- ou -A₃-A₄-A₅-A₁₂-A₁₃- (A₃ étant lié à A₀), notamment -A₃-, -A₃-A₄-A₅- avec :
▪ A₃ représentant une liaison simple, O, S, NR₃₃, -X₅-C(=X₆)-X₇-, -X₅-CH₂-X₇- ou 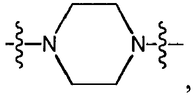
▪ A₄ et A₁₂ représentant, indépendamment l'un de l'autre, un groupe (C₁-C₆)alkyle, aryl, (C₁-C₆)alkyle-aryl ou aryl-(C₁-C₆)alkyle ; et notamment (C₁-C₆)alkyle,
▪ As représentant une liaison simple, O, S, NR₃₄, -X₈-C(=X₉)-X₁₀-, -X₈-CH₂-X₁₀- ou 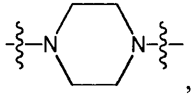
▪ A₁₃ représentant une liaison simple, O, S, NR₃₉, -X₅-C(=X₆)-X₇-, -X₅-CH₂-X₇- ou 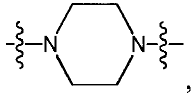
▪ X₅ à X₁₀ représentant, indépendamment les uns des autres, une liaison simple ou O, S, NR₃₅ ou 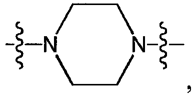 et
▪ R₃₃ à R₃₅ et R₃₉ représentant, indépendamment les uns des autres, un atome d'hydrogène ou un groupe (C₁-C₆)alkyle, et notamment un atome d'hydrogène.

Lorsque X₃ représente un groupe A₃, il pourra s'agir en particulier d'un groupe NR₃₃ ou -X₅-C(=X₆)-X₇-, et en particulier d'un groupe NH, -C(=O)-NH- ou -NH-C(=S)-NH-.

Lorsque X₃ représentera un groupe -A₃-A₄-A₅-, A₃ pourra représenter une liaison simple, O, NR₃₃ ou -X₅-C(=X₆)-X₇-, en particulier une liaison simple, O, NR₃₃, C(=O), ou- X₅-C(=O)-O- ; A₄ pourra représenter un groupe (C₁-C₆)alkyle ou aryl, en particulier un groupe phényle ou (CH₂)ₐ avec a représentant un nombre entier compris entre 1 et 5, notamment entre 1 et 3 ; et A₅ représentant -X₅-C(=X₆)-X₇-, en particulier -X₅-C(=O)-NR₃₅- tel que -C(=O)-NH- ou -O-C(=O)-NH-.

Lorsque X₃ représente un groupe -A₃-A₄-A₅-A₁₂-A₁₃-, il pourra s'agir en particulier d'un groupe -NR₃₃-A₄-C(O)O-A₁₂-C(O)O- ou -NR₃₃-A₄-C(O)O-A₁₂-C(O)NR₃₉- avec notamment R₃₃ = Me et R₃₉ = H.

X₃ représentera plus particulièrement un groupe -A₃- ou -A₃-A₄-A₅- avec :
▪ A₃ représentant une liaison simple, O, S, NR₃₃ ou -X₅-C(=X₆)-X₇-, notamment une liaison simple ou un groupe NR₃₃ ou -X₅-C(=X₆)-X₇-, tel que -X₅-C(=X₆)-NR₃₅-, et notamment tel que -C(=O)-O-, -C(=O)-NR₃₅- ou -C(=S)-NR₃₅-,
▪ A₄ étant tel que défini précédemment, par exemple -CH₂-, -CH₂-CH₂-, et
▪ A₅ représentant un groupe -X₈-C(=X₉)-X₁₀-, tel que -X₈-C(=O)-X₁₀-, notamment tel que -O-C(=O)-NR₃₅-,
X₃ représentera notamment un groupe :
▪ -A₃- représentant une liaison simple ou un groupe NR₃₃ ou -X₅-C(=X₆)-NR₃₅-, tel que -C(=X₆)-NR₃₅-, avec X₆ représentant de préférence O ou S, ou
▪ -A₃-A₄-A₅- avec :
   - A₃ représentant une liaison simple ou un groupe -X₅-C(=X₆)-X₇-, tel que -C(=O)-X₇- et notamment -C(=O)-O-,
   - A₄ tel que défini précédemment, et
   - A₅ représentant un groupe NR₃₄ ou -X₈-C(=X₉)-NR₃₅-, tel que -X₈-C(=O)-NR₃₅-. X₃ pourra représenter notamment un groupe NH, -C(=O)NH-, -C(=S)-NH- ou -C(=O)O-(CH₂)₂-OC(=O)-, ou encore -NH-C(=S)-NH-.

Le résidu R₁ des dérivés d'acide hydroxy-bisphosphonique divulgués dans la présente description peuvent être un résidu d'un principe actif utile pour le traitement ou le diagnostic d'une pathologie du remodelage osseux ostéolytique ou ostéocondensante, telle que les tumeurs osseuses primitives (telles qu'un ostéosarcome, un chondrosarcome, une tumeur à cellules géantes, ou un sarcome d'Ewing), les métastases osseuses, le myélome multiple, les dérégulations du métabolisme phospho-calcique telles que l'hypercalcémie, l'ostéoporose et les pathologies inflammatoires telles que la poly-arthrite rhumatoïde ou les descellements péri-prothétiques.

En particulier, le résidu R₁ pourra être un résidu d'une molécule d'intérêt diagnostique choisi parmi les résidus de molécules fluorescentes telles que le groupe (5-dimethylamino)naphtalene-1-sulfonyle (groupement dansyle), le groupe 7-nitro-1,2,3-benzoxadiazole (groupement NBD), un résidu de 1-pyrènecarboxaldéhyde, les résidus de la fluorescéine et de ses dérivés tels que la fluorescéine isothiocyanate (FITC) et les rhodamines telles que la rhodamine B, et les résidus des dérivés cyanines tels que les fluorescyanines et la gallocyanine ; et les résidus de molécules luminescentes telles que les résidus des dérivés de dioxetane et des sulfures alcalins ou alcalino-terreux. Les dérivés d'acide hydroxy-bisphosphonique correspondants pourront alors être utilisés pour l'imagerie du tissu osseux, notamment à des fins de diagnostic.

En particulier, le résidu R₁ pourra être choisi parmi des résidus de molécule d'intérêt thérapeutique choisie parmi des agents anticancéreux, des anti-inflammatoires, des antibiotiques, des agents antibactériens, des anesthésiques, des stéroïdes et des peptides à activité pro-formation ou anti-résorption osseuse.

Le résidu R₁ pourra être plus particulièrement issu d'une molécule d'intérêt thérapeutique choisie parmi des agents anticancéreux comme des molécules alkylantes telles que les analogues, notamment azotés, du gaz moutarde, l'ifosfamide et ses dérivés et le chlorambucil et ses dérivés, des molécules antinéoplasiques comme la doxorubicine, le cis-platine, l'adriamycine, l'actinomycine, le fluorouracil, le méthotrexate, l'étoposide, la vincristine, la podophyllotoxine, le busulphan, le docetaxel, le 5-fuoro-uracyl et leurs dérivés, ou encore des inhibiteurs de la topoisomérase 1 comme l'irinotécan ou ses analogues comme le SN38 ; des anti-inflammatoires comme des corticoïdes tels que le dexaméthasone et ses dérivés, ou des anti-inflammatoires non-stéroïdiens tels que l'ibuprofène, l'indométacine, le bindazac, l'acide étodolique, le diclofenac, le lonazolac, et leurs dérivés ; des antibiotiques tels que la spiramycine ; des agents antibactériens tels que le salicylaldéhyde et ses dérivés tels que le dalyde ; des anesthésiques tels que la benzocaïne ; et des stéroïdes comme les dérivés de l'oestradiol et de l'oestrone (encore appelée estrone).

R₁ pourra être notamment un résidu de podophyllotoxine, d'analogue azotée de gaz moutarde, de chlorambucil, de méthotrexate, de doxorubicine, de SN38, d'ibuprofène, de diclofenac, d'oestrone, de spiramycine, de dalyde ou de benzocaïne.

R₁ pourra être notamment un résidu de podophyllotoxine, d'analogue azotée de gaz moutarde, de méthotrexate, d'ibuprofène, de diclofenac, d'oestrone, de spiramycine ou de benzocaïne ; et en particulier un résidu de podophyllotoxine ou d'analogue azotée de gaz moutarde.

R₁ pourra être également un groupe dansyle, un groupe 7-nitro-1,2,3-benzoxadiazole, un résidu de 1-pyrènecarboxaldéhyde, de fluorescéine ou de ses dérivés ou d'une rhodamine, notamment rhodamine B ; et en particulier un groupe dansyle, un résidu de 1-pyrènecarboxaldéhyde ou de fluorescéine ou de ses dérivés.

La présente description divulgue également des composés de formule (la) suivante : 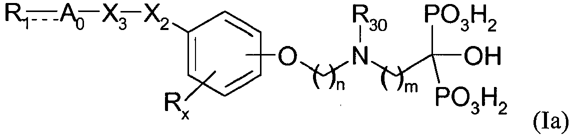 pour laquelle -̅-̅-̅-A₀, R₁, R₃₀, X₂, X₃, n et m sont tels que définis précédemment et Rₓ représente un ou plusieurs substituants du noyau phényle choisis dans le groupe constitué par un atome d'hydrogène, un atome d'halogène, NO₂, -CN, -OH, -SH, -NR₁₂R₁₃, -B(OH)₂, -SO₃R₁₄, -COOR₁₅, -C(O)ONR₁₆R₁₇, -OPH(O)OR₁₈, -PH(O)OR₁₉, -OP(O)(OR₂₀)(OR₂₁), -P(O)(OR₂₂)(OR₂₃), -C(O)R₂₄, -PR₂₅R₂₆, (C₁-C₆)alkyle et (C₁-C₆)alcoxy, avec R₁₂ à R₂₄ représentant, indépendamment les uns des autres, un atome d'hydrogène ou un groupe (C₁-C₆)alkyle et R₂₅ et R₂₆ représentant, indépendamment l'un de l'autre, un groupe (C₁-C₆)alkyle.

L'oxygène sera notamment en position méta ou para, et de préférence méta, sur le noyau phényle par rapport au groupe X₂.

R₃₀ représentera plus particulièrement un groupe (C₁-C₆)alkyle, tel que méthyle. n pourra représenter 3 et m pourra représenter 2.

Rₓ représentera plus particulièrement un ou plusieurs, et notamment un seul, substituants du noyau phényle choisis dans le groupe constitué par un atome d'hydrogène, un atome d'halogène, NO₂ et (C₁-C₆)alcoxy, et notamment choisis dans le groupe constitué par un atome d'hydrogène et NO₂.

Rₓ représentera notamment un atome d'hydrogène ou un groupe NO₂. Il pourra être avantageusement en position ortho sur le noyau phényle par rapport à l'oxygène.

La présente invention concerne des dérivés d'acide hydroxy-bisphosphonique bifonctionnel choisi parmi : 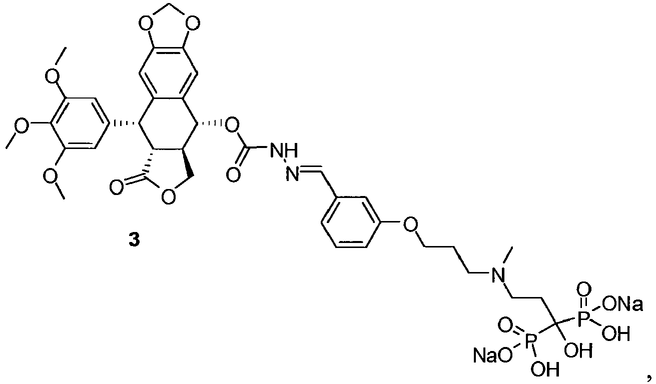 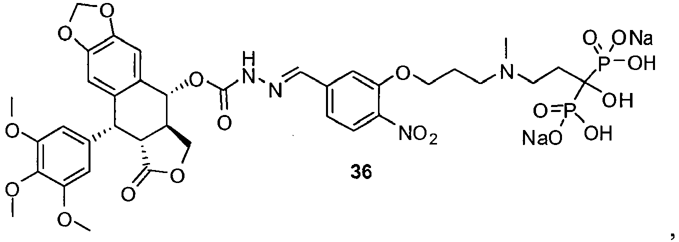 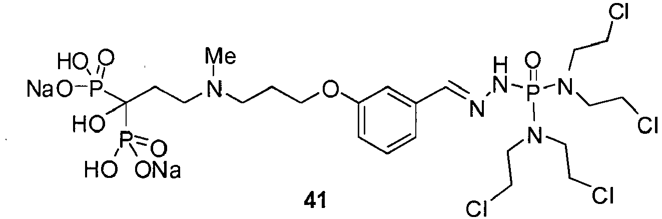 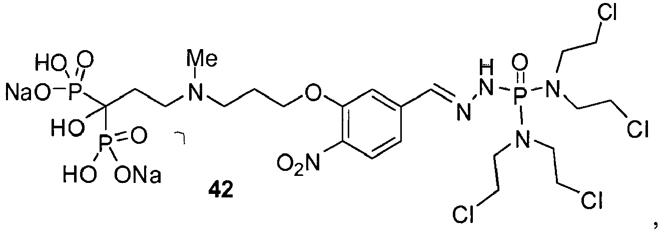 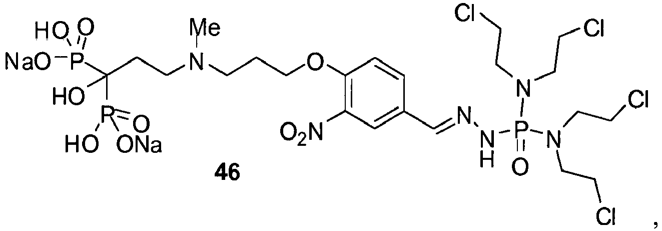 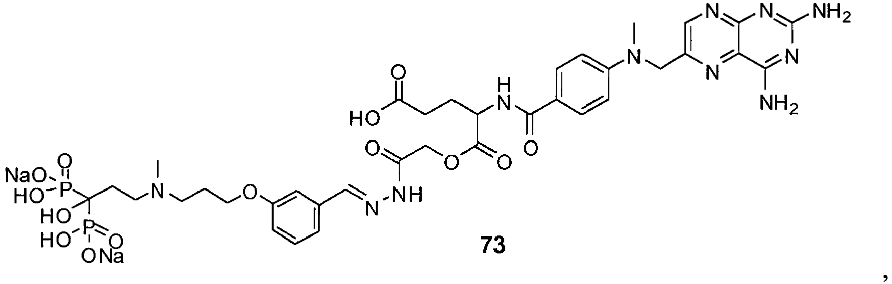 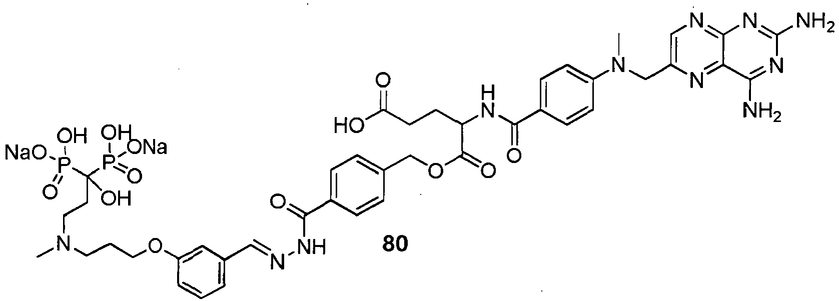 et 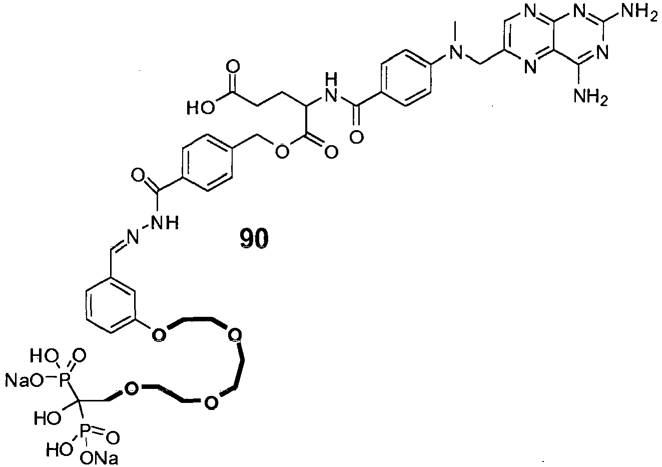 ou un sel pharmaceutiquement acceptable de celui-ci.

La présente invention concerne également un dérivé d'acide hydroxy-bisphosphonique tel que décrit ci-dessus ou un sel pharmaceutiquement acceptable de celui-ci, pour son utilisation en tant que médicament, notamment pour cibler le tissu osseux.

La présente invention concerne également l'utilisation d'un dérivé d'acide hydroxy-bisphosphonique de l'invention ou d'un sel pharmaceutiquement acceptable de celui-ci, pour la préparation d'une composition pharmaceutique, ciblant plus particulièrement le tissu osseux.

En particulier, les dérivés d'acide hydroxy-bisphosphonique de l'invention pourront être utilisés en tant que médicament destiné au traitement d'une pathologie du remodelage osseux ostéolytique ou ostéocondensante telle que les tumeurs osseuses primitives comme un ostéosarcome, un chondrosarcome, une tumeur à cellules géantes ou un sarcome d'Ewing ; les métastases osseuses ; le myélome multiple.

Avantageusement, les dérivés d'acide hydroxy-bisphosphonique sont utilisés dans un traitement antitumoral, notamment pour traiter l'hypercalcémie maligne, les tumeurs osseuses primitives telles que l'ostéosarcome et les métastases osseuses.

La présente description décrit également un dérivé d'acide hydroxy-bisphosphonique tel que décrit ci-dessus ou un sel pharmaceutiquement acceptable de celui-ci, pour son utilisation en tant que produit de diagnostic, notamment pour cibler le tissu osseux, ou encore l'utilisation d'un dérivé d'acide hydroxy-bisphosphonique ou d'un sel pharmaceutiquement acceptable de celui-ci, pour la préparation d'une composition diagnostique, ciblant plus particulièrement le tissu osseux.

Dans ce cadre, les dérivés d'acide hydroxy-bisphosphonique divulgués dans la présente description peuvent être utilisés en tant que produit de diagnostic pour imagerie du tissu osseux destiné notamment à diagnostiquer une pathologie du remodelage osseux ostéolytique ou ostéocondensante telle que les tumeurs osseuses primitives comme un ostéosarcome, un chondrosarcome, une tumeur à cellules géantes ou un sarcome d'Ewing ; les métastases osseuses ; le myélome multiple ; les dérégulations du métabolisme phospho-calcique comme l'hypercalcémie ; l'ostéoporose ; et les pathologies inflammatoires comme la polyarthrite rhumatoïde ou les descellements péri-prothétiques.

Les dérivés d'acide hydroxy-bisphosphonique divulgués dans la description sont également décrits pour leur utilisation dans le traitement de l'ostéoporose ou dans un traitement anti-inflammatoire, notamment pour traiter la polyarthrite rhumatoïde.

La présente description divulgue par ailleurs une composition diagnostique comprenant au moins un dérivé d'acide hydroxy-bisphosphonique de la présente description, tel que décrit précédemment, ou un sel pharmaceutiquement acceptable de celui-ci et au moins un véhicule pharmaceutiquement acceptable.

La présente invention concerne par ailleurs une composition pharmaceutique comprenant au moins un dérivé d'acide hydroxy-bisphosphonique de l'invention, tel que décrit précédemment, ou un sel pharmaceutiquement acceptable de celui-ci et au moins un véhicule pharmaceutiquement acceptable.

Cette composition pourra être formulée de manière à permettre son administration, notamment, par voie sous-cutanée, intra-veineuse, intramusculaire ou transdermique, c'est-à-dire de préférence sous forme d'une solution injectable ou sous forme d'un patch, et destinée aux mammifères, y compris l'homme. La posologie variera selon le traitement et selon l'affection en cause.

Les composés de l'invention en tant que principes actifs peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier peut se rendre compte lui-même.

La présente invention a également pour objet une composition pharmaceutique telle que définie ci-dessus pour son utilisation en tant que médicament. En particulier, les compositions de l'invention pourront être utilisées pour le traitement d'une pathologie du remodelage osseux ostéolytique ou ostéocondensante, telle que les tumeurs osseuses primitives (telles qu'un ostéosarcome, un chondrosarcome, une tumeur à cellules géantes ou un sarcome d'Ewing), les métastases osseuses, le myélome multiple. La présente description divulgue également une composition diagnostique telle que définie ci-dessus pour son utilisation en tant que produit de diagnostic.

Les compositions thérapeutiques peuvent être utilisées dans un traitement antitumoral, notamment pour traiter l'hypercalcémie maligne, les tumeurs osseuses primitives et les métastases osseuses.

Dans le cadre de la présente description, les compositions diagnostiques sont utilisées pour l'imagerie du tissu osseux.

Des compositions thérapeutiques utilisées dans le traitement de l'ostéoporose ou dans un traitement anti-inflammatoire, notamment pour traiter la polyarthrite rhumatoïde sont aussi décrites.

Les composés de formule (I) peuvent être préparés selon les étapes successives suivantes :
(a1) couplage entre une composé de formule (II) suivante : 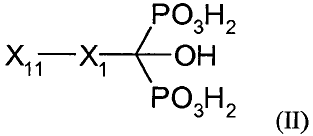 pour laquelle X₁ est tel que défini ci-dessus et X₁₁ représente une fonction -CHO, ou -NH₂,
   avec un composé de formule (III) suivante : 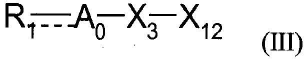 pour laquelle -̅-̅-̅-A_{0,} R₁ et X₃ sont tels que définis précédemment et X₁₂ représente une fonction -NH₂ lorsque X₁₁ = CHO et représente une fonction -CHO lorsque X₁₁ = NH₂,
   pour donner un composé de formule (I),
(b1) éventuellement salification du composé de formule (I) obtenu à l'étape (a1) précédente pour donner un sel pharmaceutiquement acceptable de celui-ci, et
(c1) séparation du milieu réactionnel du composé de formule (I) ou d'un de ses sels pharmaceutiquement acceptables obtenu à l'étape (a1) ou (b1) précédente.

### Etape (a1) :

La réaction de couplage entre l'aldéhyde (CHO) et l'amine (NH₂), pour donner une fonction imine, peut être effectuée dans un solvant polaire tel que de l'eau, MeOH, EtOH, iPrOH, PrOH, tBuOH, BuOH, DMF, DMSO, MeNO2, MeCN, THF, dioxane, pyridine, HMPT, diglime, éthylène glycol, glycérol, et leurs mélanges et notamment tel que de l'eau, du méthanol et leurs mélanges.

Une base peut éventuellement être ajoutée au milieu réactionnel, telle que NaOH, KOH, LiOH, Ca(OH)₂, NaHCO₃, Na₂CO₃, KHCO₃, K₂CO₃, Li₂CO₃, NH₃, une amine, pyridine, picoline, quinoléine, DMAP, DBU, etc., et notamment telle que NaOH ou KOH.

Un acide peut au contraire être ajouté tel que HCl, H₂SO₄, AcOH, CF₃COOH, ou encore HCOOH.

La réaction pourra notamment être réalisée à température ambiante, c'est-à-dire à une température comprise entre 15 et 40°C, notamment entre 20 et 30°C, et en particulier environ 25°C.

Toutefois, si les réactifs ne sont pas sensibles à la chaleur, le milieu réactionnel peut être chauffé jusqu'à environ 100-150°C.

Des étapes supplémentaires de protection et déprotection peuvent être utilisées dans le cadre de ce procédé, si nécessaire, notamment dans le cas où le principe actif posséderait d'autres fonctionnalités pouvant réagir dans les conditions de la réaction.

### Etape (b1) :

L'étape de salification sera réalisée en présence d'un acide ou d'une base pharmaceutiquement acceptable tel que défini ci-dessus. Il pourra s'agir en particulier d'une base telle que NaOH, KOH, LiOH, Ca(OH)₂, NaHCO₃, Na₂CO₃, KHCO₃, K₂CO₃, Li₂CO₃, NH₃, une amine, pyridine, picoline, quinoléine, DMAP, DBU, etc., notamment telle que NaOH ou KOH, et en particulier NaOH.

### Etape (c1) :

Le composé de formule (I) ainsi obtenu pourra être séparé du milieu réactionnel par des méthodes bien connues de l'homme du métier, comme par exemple par extraction, évaporation du solvant ou encore par précipitation et filtration.

Par ailleurs, ce composé pourra être purifié si nécessaire par des techniques bien connues de l'homme du métier, comme par recristallisation si le composé est cristallin, par chromatographie sur colonne sur gel de silice ou en phase inverse ou encore par chromatographie liquide haute performance (HPLC).

Les composés de formule (II) pourront être préparés par des techniques bien connues de l'homme du métier.

Lorsque A₂ = NR₃₀, le composé de formule (II) peut être préparé à partir d'un composé de formule (IV) suivant :

X₁₁-X₄-A₁-H (IV)

pour laquelle X₄, X₁₁ et A₁ sont tels que définis précédemment
selon les étapes successives suivantes :
(a2) éventuellement protection de la fonction X₁₁ pour donner un composé de formule (IVbis) suivante :

   Xbis₁₁-X₄-A₁-H (IVbis)

   pour laquelle X₄ et A₁ sont tels que définis précédemment et Xbis₁₁ représente une fonction CHO ou NH₂ protégée,
(b2) réaction du composé de formule (IV) ou (IVbis) avec un composé de formule (V) suivante :

   A₆-(CH₂)ₙ-A₇ (V)

   pour laquelle n est tel que défini précédemment et A₆ et A₇ représentent, indépendamment l'un de l'autre, un groupement partant tel qu'un atome d'halogène, et notamment un atome de brome, un tosylate ou encore un mésylate,
   pour donner un composé de formule (VI) ou (VIbis) suivante :

   X₁₁-X₄-A₁-(CH₂)ₙ-A₇ (VI)

   ou

   Xbis₁₁-X₄-A₁-(CH₂)ₙ-A₇ (VIbis)

   pour lequel X₄, X₁₁, Xbis₁₁, A₁, A₇ et n sont tels que définis précédemment,
(c2) réaction du composé de formule (VI) ou (VIbis) précédente avec une amine de formule R₃₀NH₂, R₃₀ étant tel que défini précédemment pour donner un composé de formule (VII) ou (VIIbis) suivante :

   X₁₁-X₄-A₁-(CH₂)ₙ-NHR₃₀ (VII)

   ou

   Xbis₁₁-X₄-A₁-(CH₂)ₙ-NHR₃₀ (VIIbis)

   pour lequel X₄, X₁₁, Xbis₁₁, A₁, R₃₀ et n sont tels que définis précédemment,
(d2) réaction du composé de formule (VII) ou (VIIbis) précédente avec un acide de formule A₈-(CH₂)ₘCOOH pour lequel A₈ représente un groupe partant tel qu'un atome d'halogène, un tosylate ou un mésylate, ou avec l'acide acrylique lorsque m = 2 pour donner un composé de formule (VIII) ou (VIIIbis) suivante :

   X₁₁-X₄-A₁-(CH₂)ₙ-NR₃₀-(CH₂)ₘ-COOH (VIII)

   ou

   Xbis₁₁-X₄-A₁-(CH₂)ₙ-NR₃₀-(CH₂)ₘ-COOH (VIIIbis)

   pour lequel X₄, X₁₁, Xbis₁₁, A₁, R₃₀, n et m sont tels que définis précédemment,
(e2) conversion de la fonction acide du composé de formule (VIII) ou (VIIIbis) précédente en fonction acide hydroxybisphosphonique pour donner un composé de formule (II) ou un composé de formule (IIbis) suivante : 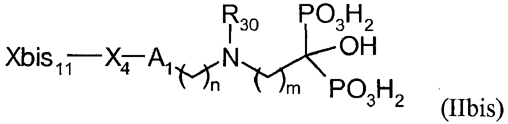 pour lequel X₄, Xbis₁₁, A₁, R₃₀, n et m sont tels que définis précédemment,
(f2) éventuellement déprotection de la fonction Xbis₁₁ pour donner un composé de formule (II), et
(g2) séparation du milieu réactionnel du composé de formule (II) obtenu à l'étape (e2) ou (f2) précédente.

### Etape (a2) :

Les composés de formule (IV) sont des composés soit commerciaux, soit accessibles par des techniques bien connues de l'homme du métier.

La fonction aldéhyde (CHO) pourra être protégée par tout groupe protecteur bien connu de l'homme du métier et notamment sous forme d'acétal cyclique ou non de formule -C(OR₃₆)(OR₃₇) où R₃₆ et R₃₇, identiques ou différents, de préférence identiques, représentent un groupement (C₁-C₆)alkyle ou R₃₅ et R₃₇ forment ensemble une chaîne de formule -(CH₂)ₚ- avec p représentant 2 ou 3, notamment 3.

La fonction aldéhyde pourra ainsi être protégée sous forme d'acétal par réaction avec l'alcool correspondant, R₃₆OH et R₃₇OH, de préférence identiques, ou, lorsque R₃₆ et R₃₇ forment ensemble une chaîne avec HO-(CH₂)ₚ-OH.

La fonction amine (NH2) pourra être protégée par tout groupe protecteur bien connu de l'homme du métier et notamment par un groupement Boc (butyloxycarbonyle).

### Etape (b2) :

Par « groupe partant », on entend, au sens de la présente invention, un groupement chimique qui peut être facilement déplacé par un nucléophile lors d'une réaction de substitution nucléophile, le nucléophile étant plus particulièrement une amine, et notamment une amine secondaire. Un tel groupe partant peut être plus particulièrement un atome d'halogène tel qu'un atome de chlore, un mésylate (MsO-) ou encore un tosylate (*p*-Me-Ph-O-).

Cette étape pourra être réalisée en présence d'une base telle que K₂CO₃. A₆ et A₇ pourront représenter chacun un atome d'halogène tel qu'un atome de brome.

### Etape (c2) :

Cette étape pourra être réalisée dans un solvant tel que le THF, notamment à température ambiante.

### Etape (d2) :

Cette étape pourra être réalisée en présence d'une base telle que DIPEA, notamment dans un solvant tel qu'un alcool, par exemple le méthanol, et notamment à température ambiante.

### Etape (e2) :

Au cours de cette étape, la fonction acide carboxylique pourra être activée, notamment sous forme de chlorure d'acide (-COCl) ou encore de dérivé borane par réaction avec un borane tel que le catécholborane.

La fonction acide carboxylique activée est alors mise à réagir avec du tris(triméthylsilyl)phosphite puis avec un alcool aliphatique tel que le méthanol pour donner la fonction acide hydroxybisphosphonique.

### Etape (f2) :

Les fonctionnalités (aldéhyde ou amine) sous forme protégée sont déprotégées par des techniques bien connues de l'homme du métier, notamment par traitement acide ou basique. Les dérivés acétals pourront notamment être déprotégés pour libérer la fonction aldéhyde en milieu acide, notamment en présence d'HCl.

### Etape (g2) : voir étape (c1) précédente.

La présente description divulgue également un composé de formule (II) suivante : 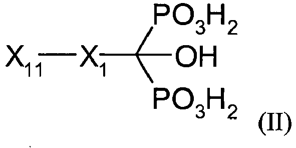 pour laquelle
- X₁ représente une chaîne -X₄-A₁-(CH₂)ₙ-A₂-(CH₂)ₘ-,
- X₁₁ représente un groupe -CHO ou -NH₂, et notamment -CHO,
- X₄ représente une liaison simple ou un groupe aryle ou hétéroaryle éventuellement substitué,
- A₁ représente une liaison simple, O, S, NR₂₇, -C(O)-, -C(S)-, -C=N-, -N=C-, -C=C-, -C≡C-, -OC(O)-, -C(O)O-, -SC(O)-, -C(O)S-, -N(R₂₈)C(O)- ou -C(O)N(R₂₉)-,
- A₂ représente une liaison simple, O, S, NR₃₀, -C(O)-, -C(S)-, -C=N-, -N=C-, -C=C-, -C≡C-, -OC(O)-, -C(O)O-, -SC(O)-, -C(O)S-, -N(R₃₁)C(O)-, -C(O)N(R₃₂)-, -O-(CH₂CH₂O)ₖ- ou un groupe -A₉-(CH₂)ₐ-A₁₀-CH₂)_{b}-A₁₁-,
- A₉ et A₁₁ représentent chacun, indépendamment l'un de l'autre, O, S, ou NR₃₈,
- A₁₀ représente un groupe aryle, hétéroaryle ou hétérocyclique,
- n représente un nombre entier compris entre 0 et 5, notamment compris entre 1 et 5, et notamment 3,
- m représente un nombre entier compris entre 0 et 5, notamment compris entre 1 et 5, et notamment 2,
- k représente un nombre entier compris entre 1 et 10, notamment compris entre 1 et 5,
- a et b représentent, indépendamment l'un de l'autre, un nombre entier compris entre 0 et 5,
- R₂₇ à R₂₉ représentent, indépendamment les uns des autres, un atome d'hydrogène ou un groupe (C₁-C₆)-alkyle, et
- R₃₀ à R₃₂ et R₃₈ représentent, indépendamment les uns des autres, un atome d'hydrogène ou un groupe (C₁-C₆)-alkyle, cycloalkyle, hétérocyclique, aryle, hétéroaryle ou acyle, et de préférence un atome d'hydrogène ou un groupe (C₁-C₆)-alkyle ou aryle, et encore de préférence un atome d'hydrogène ou un groupe (C₁-C₆)-alkyle tel que méthyle,
ou un sel pharmaceutiquement acceptable de celui-ci.

La présente description divulgue des composés de formule (II) dans laquelle :
- X₁ représente une chaîne -X₄-A₁-(CH₂)ₙ-A₂-(CH₂)ₘ-,
- X₁₁ représente un groupe -CHO ou -NH₂, et notamment -CHO,
- X₄ représente un groupe aryle ou hétéroaryle éventuellement substitué,
- A₁ représente une liaison simple, O, S, NR₂₇, -C(O)-, -OC(O)-, -C(O)O-, -SC(O)-, -C(O)S-, -N(R₂₈)C(O)- ou -C(O)N(R₂₉)-,
- A₂ représente O, S, NR₃₀, -C(O)-, -OC(O)-, -C(O)O-, -SC(O)-, -C(O)S-, -N(R₃₁)C(O)- ou -C(O)N(R₃₂)-,
- n représente un nombre entier compris entre 1 et 5, et notamment 3,
- m représente un nombre entier compris entre 0 et 5, notamment compris entre 1 et 5, et notamment 2,
- R₂₇ à R₂₉ représentent, indépendamment les uns des autres, un atome d'hydrogène ou un groupe (C₁-C₆)-alkyle, et
- R₃₀ à R₃₂ représentent, indépendamment les uns des autres, un atome d'hydrogène ou un groupe (C₁-C₆)-alkyle, cycloalkyle, hétérocyclique, aryle, hétéroaryle ou acyle, et de préférence un atome d'hydrogène ou un groupe (C₁-C₆)-alkyle ou aryle, et encore de préférence un atome d'hydrogène ou un groupe (C₁-C₆)-alkyle tel que méthyle,
ou un sel pharmaceutiquement acceptable de celui-ci.

La présente description divulgue des composés de formule (II) dans laquelle X₁ ne représente pas une liaison simple.

La présente description divulgue des composés de formule (II) dans laquelle X₄ représente un groupe aryle ou hétéroaryle éventuellement substitué, et notamment un groupe aryle, tel que phényle, éventuellement substitué.

La présente description divulgue des composés de formule (II) dans laquelle X₄ représente un groupe phényle, naphtyle ou indolyle éventuellement substitué, le groupe indolyle étant de préférence lié à A₁ par l'intermédiaire de son atome d'azote, et de préférence un groupe phényle éventuellement substitué.

Les groupes aryle et hétéroaryle, tels que phényle, naphtyle et indolyle, de X₄ pourront être substitués comme défini précédemment, et notamment par un ou plusieurs groupements choisis dans le groupe constitué par un atome d'halogène, NO₂ et (C₁-C₆)alcoxy, et notamment NO₂.

La présente description divulgue des composés de formule (II) dans laquelle A₁ représente une liaison simple, O, S, NR₂₇, -C(O)-, -OC(O)-, -C(O)O-, -SC(O)-, -C(O)S-, -N(R₂₈)C(O)- ou -C(O)N(R₂₉)-.

La présente description divulgue des composés de formule (II) dans laquelle A₁ représente une liaison simple, O, S ou NR₂₇, notamment une liaison simple ou O, et de préférence O.

La présente description divulgue des composés de formule (II) dans laquelle A₁ représente une liaison simple quand X₄ représente un groupe hétéroaryle, tel qu'un groupe indolyle, comprenant un atome d'azote et lié à A₁ par l'intermédiaire de cet atome d'azote. A₁ sera différent d'une liaison simple, et notamment représentera un atome d'oxygène, quand X₄ représentera un aryle, tel qu'un groupe phényle ou naphtyle, ou un hétéroaryle lié à A₁ par l'intermédiaire d'un atome de carbone.

La présente description divulgue des composés de formule (II) dans laquelle A₂ représente O, S, NR₃₀, -C(O)-, -OC(O)-, -C(O)O-, -SC(O)-, -C(O)S-, -N(R₃₁)C(O)- ou -C(O)N(R₃₂)-.

La présente description divulgue des composés de formule (II) dans laquelle A₂ représente O, S ou NR₃₀, et de préférence NR₃₀, avec R₃₀ tel que défini précédemment et représentant notamment un atome d'hydrogène ou un groupe (C₁-C₆)-alkyle ou aryle, et de préférence un atome d'hydrogène ou un groupe (C₁-C₆)-alkyle tel que méthyle.

La présente description divulgue des composés de formule (II) dans laquelle A₂ représente un groupe NR₃₀ avec R₃₀ représentant un atome d'hydrogène ou un groupe (C₁-C₆)-alkyle, et de préférence un groupe (C₁-C₆)-alkyle tel que méthyle.

La présente description divulgue des composés de formule (II) dans laquelle X₁ représente une chaîne de formule -X₄-A₁-(CH₂)ₙ-A₂-(CH₂)ₘ- avec :
- X₄ représentant un groupe phényle, éventuellement substitué comme défini précédemment et notamment par NO₂,
- A₁ représentant un atome d'oxygène,
- A₂ représentant un groupe NR₃₀ avec R₃₀ représentant un groupe (C₁-C₆)-alkyle tel que méthyle,
- n représentant 3, et 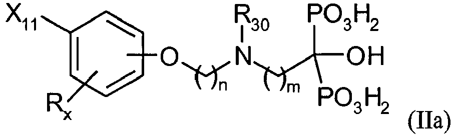
pour laquelle R₃₀, X₁₁, Rₓ, n et m sont tels que définis précédemment.

Les composés de formule (II) peuvent être préparés par des techniques bien connues de l'homme du métier.

Lorsque A₂ = NR₃₀, le composé de formule (II) peut être préparé par le procédé indiqué ci-dessus, avec éventuellement une étape de salification le cas échéant, avant ou après la dernière étape de séparation du milieu réactionnel du composé préparé.

La présente invention concerne plus particulièrement un composé de formule (II) choisi parmi : 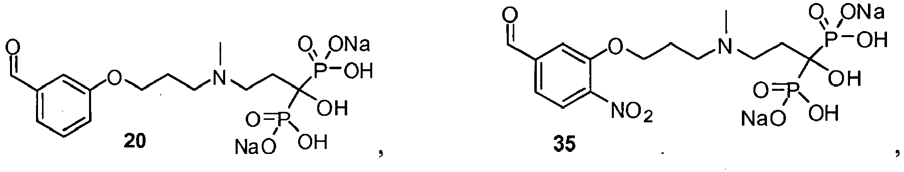 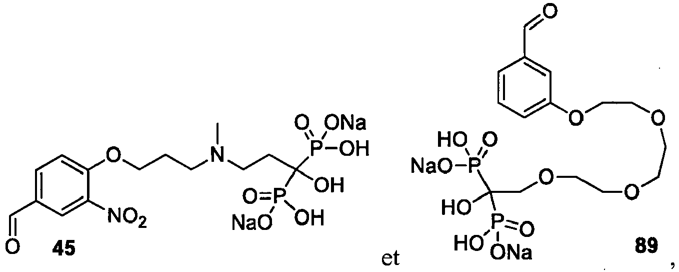 ou un sel pharmaceutiquement acceptable de celui-ci.

La présente description divulgue également l'utilisation d'un composé de formule (II) telle que définie ci-dessus pour préparer une molécule d'intérêt thérapeutique ou diagnostique vectorisée, notamment destinée à cibler le tissu osseux.

La molécule d'intérêt thérapeutique ou diagnostique, éventuellement fonctionnalisée pour avoir une fonctionnalité X₁₂ = CHO ou NH₂, est alors lié au composé de formule (II) par couplage entre sa fonctionnalité X₁₂ et la fonctionnalité X₁₁ du composé de formule (II) (par couplage entre CHO et NH₂).

La molécule d'intérêt thérapeutique ou diagnostique, éventuellement fonctionnalisée pour avoir une fonctionnalité X₁₂ = CHO ou NH₂, est alors lié au composé de formule (II) par couplage entre sa fonctionnalité X₁₂ et la fonctionnalité X₁₁ du composé de formule (II) (par couplage entre CHO et NH₂).

La molécule d'intérêt thérapeutique ou diagnostique pourra être une molécule utile pour le traitement ou le diagnostic d'une pathologie du remodelage osseux ostéolytique ou ostéocondensante, telle que les tumeurs osseuses primitives (telles qu'un ostéosarcome, un chondrosarcome, une tumeur à cellules géantes, ou un sarcome d'Ewing), les métastases osseuses, le myélome multiple, les dérégulations du métabolisme phospho-calcique telles que l'hypercalcémie, l'ostéoporose et les pathologies inflammatoires telles que la poly-arthrite rhumatoïde ou les descellements péri-prothétiques.

La molécule d'intérêt diagnostique pourra être plus particulièrement une molécule utile pour l'imagerie médicale, notamment du tissu osseux, telle qu'une molécule fluorescente comme le résidu (5-dimethylamino)naphtalene-1-sulfonyle (groupement dansyle), le résidu 7-nitro-1,2,3-benzoxadiazole (groupement NBD), la 1-pyrènecarboxaldéhyde, la fluorescéine et ses dérivés tels que la fluorescéine isothiocyanate (FITC) et les rhodamines telles que la rhodamine B, et les dérivés cyanines tels que les fluorescyanines et la gallocyanine ; ou une molécule luminescente comme des dérivés de dioxetane et des sulfures alcalins ou alcalino-terreux.

La molécule d'intérêt thérapeutique pourra être plus particulièrement un principe actif choisi parmi des agents anticancéreux, des anti-inflammatoires, des antibiotiques, des anesthésiques, des stéroïdes et des peptides à activité pro-formation ou anti-résorption osseuse.

La molécule d'intérêt thérapeutique pourra être choisie en particulier parmi des agents anticancéreux comme des molécules alkylantes telles que les analogues, notamment azotés, du gaz moutarde et l'ifosfamide et ses dérivés, ou des molécules antinéoplasiques comme la doxorubicine, le cis-platine, l'adriamycine, l'actinomycine, le fluorouracil, le méthotrexate, l'étoposide, la vincristine, la podophyllotoxine, le busulphan, le docetaxel, le 5-fuoro-uracyl et leurs dérivés ; des anti-inflammatoires comme des corticoïdes tels que le dexaméthasone et ses dérivés, ou des anti-inflammatoires non-stéroïdiens tels que l'ibuprofène, l'indométacine, le bindazac, l'acide étodolique, le diclofenac, le lonazolac, et leurs dérivés ; des antibiotiques tels que la spiramycine ; des anesthésiques tels que la benzocaïne ; des stéroïdes comme les dérivés de l'oestradiol et de l'oestrone.

La molécule d'intérêt thérapeutique pourra être notamment la podophyllotoxine ou un analogue azoté de gaz moutarde.

La molécule d'intérêt diagnostique pourra être un dérivé de dansyle ou de pyrène.

La molécule d'intérêt thérapeutique ou diagnostique vectorisée pourra être plus particulièrement une molécule de formule (I) telle que définie ci-dessus.

### DESCRIPTIF DES FIGURES ANNEXEES :

La figure 1 représente le pourcentage de cellules vivantes des lignées cellulaires POS1 et L929 en fonction de la concentration du composé 3 testé.
Les figures 2a et 2b représentent des images obtenues par imagerie médicale par fluorescence du dos ou du ventre respectivement de souris après injection du véhicule seul (contrôle négatif), du composé 49 de référence ou du composé 50 de référence.
La figure 3 représente l'évolution dans le temps de la libération de la molécule 114 à partir du composé 115 de référence dans un broyat de tumeur.
La figure 4 représente l'évolution dans le temps de la libération du méthotréxate (MTX) à partir du composé 80 selon l'invention dans un broyat de tumeur.
La figure 5 représente les résultats d'activité anticancéreuse sur les lignées POS1 et L929 en fonction de la concentration du composé 3 selon l'invention.
La figure 6 représente les résultats d'activité anticancéreuse sur les lignées POS1 et L929 en fonction de la concentration du composé 21 de référence.
La figure 7 représente les résultats d'activité anticancéreuse sur la lignée POS1 en fonction de la concentration du composé 80 selon l'invention (MTX vectorisé) ou du méthotréxate (MTX).
La figure 8 représente les résultats d'activité anticancéreuse sur la lignée SaOS2 en fonction de la concentration du composé 80 selon l'invention (MTX vectorisé) ou du méthotréxate (MTX).
La figure 9 représente les résultats d'activité anticancéreuse sur les lignées POS1 et L929 en fonction de la concentration du composé (III) de WO 2009/083614.

### ABREVIATIONS UTILISEES :

- CCM: Chromatographie sur Couche Mince
- CDI: Carbonyle diimidazole
- DBU: Diaza(1,3)bicyclo[5.4.0]undécane
- DCM: Dichlorométhane
- DIPEA: Diisopropyléthylamine
- DMAP: Diméthylaminopyridine
- DMF: Diméthylformamide
- DMSO: Diméthylsulfoxyde
- EDCI: 1-Ethyl-3-(3-diméthylaminopropyl)carbodiimide
- éq.: Equivalent
- ES: Electrospray
- HMDS: Hexaméthyldisilazane
- HMPT: Hexa-méthylphosphoramide
- HOBt: 1-Hydroxybenzotriazole
- PTSA: Acide *para*-toluènesulfonique
- Py: Pyridine
- RMN: Résonance Magnétique Nucléaire
- TA: Température Ambiante
- TEA: Triéthylamine
- Tf: Triflate
- THF: Tétrahydrofurane

### EXEMPLES :

### 1. Synthèse des composés

### 1.1. Synthèse des molécules de formule (II)

Le composé 20 a été préparé selon le schéma réactionnel suivant :

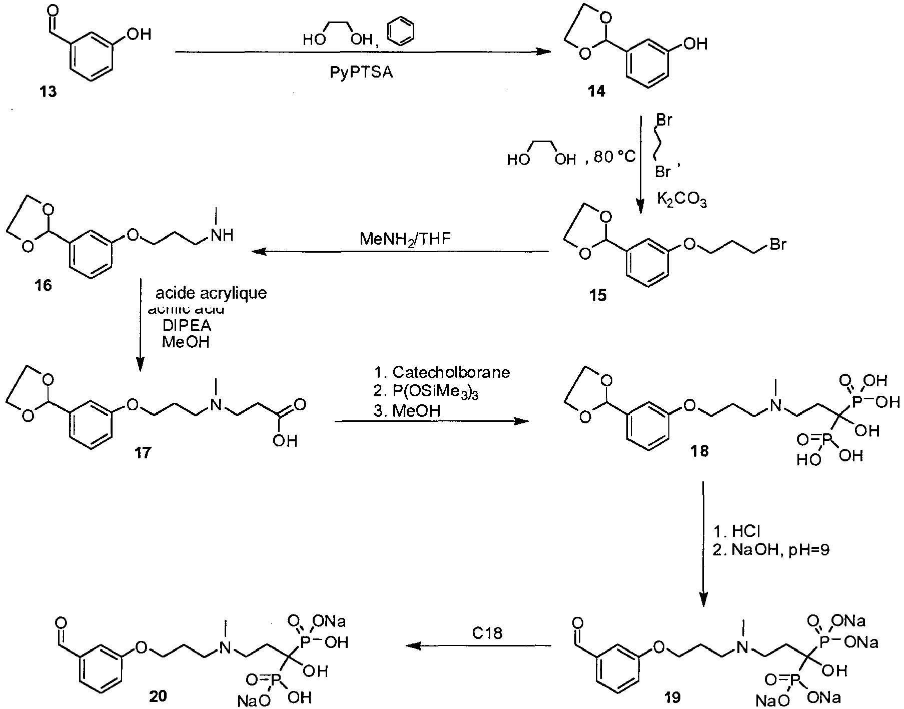

### Composé 14 :

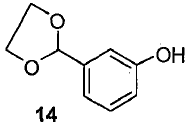

Une solution de **13** (5 g, 41 mmol, 1 éq.), de pyridine (176 mg, 2.2 mmol, 0,05 éq.) et de PTSA·H₂O (429 mg, 2.2 mmol, 0.05 éq.) dans l'éthylène glycol (100 ml) a été agitée à 80°C pendant 1 h. La réaction a été suivie par CCM. Le mélange réactionnel contenant **14** a été utilisé dans l'étape suivante sans aucun traitement. La molécule a été caractérisée par son spectre RMN 1H.

### Composé 15 :

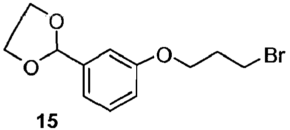

Du K₂CO₃ (6.5 g, 47 mmol, 1.15 éq.) puis du 1,3-dibromopropane (16.6 ml, 1.64 mmol, 4 éq.) ont été rajoutés au mélange réactionnel de l'étape précédente toujours à 80°C. Après 2 h d'agitation à la même température, le mélange réactionnel a été refroidi. 100 ml d'eau ont été rajoutés. La solution obtenue a été extraite avec de l'éther et la phase organique a été séchée avec Na₂SO₄ anhydre. Après concentration à pression réduite (25 mbar, 90°C), le composé **15** brut (12 g) a été obtenu sous forme d'huile orange qui a été utilisée dans l'étape suivante sans purification supplémentaire. La molécule a été caractérisée par son spectre RMN 1H.

### Composé 16 :

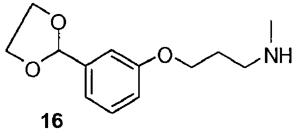

La solution de **15** brut (∼41 mmol, 1 éq.) de l'étape précédent dans MeNH₂ (2M/THF) a été laissée à TA pour 16 h. Le mélange réactionnel a été concentré à pression réduite et chromatographié sur silice (gradient de DCM à DCM : MeOH=5 :1). Ainsi, 6,9 g de **16** a été obtenu avec un rendement de 71% sur trois étapes à partir de **13.** La molécule a été caractérisée par son spectre RMN 1H.

### Composé 17 :

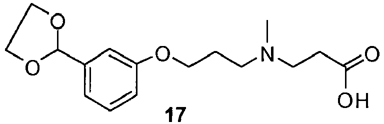

Une solution de **16** (6.9 g, 29 mmol, 1 éq.) dans un mélange de MeOH (120 ml), de DIPEA (23.9 ml, 145 mmol, 5 éq.) et d'acide acrylique (7.7 ml, 116 mmol, 4 éq.) a été laissée à TA pendant 3 jours. Le mélange réactionnel a été concentré à pression réduite à 40°C et chromatographié sur silice (gradient de DCM à MeOH). Ainsi, 4,4 g de 17 a été obtenu avec un rendement de 49%. La molécule a été caractérisée par son spectre RMN 1H.

### Composé 20 :

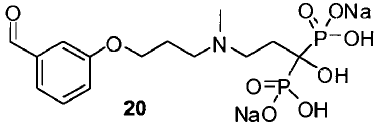

A une solution de **17** (730 mg, 2.36 mmol, 1 éq.) dans le THF (5.5 ml), du catécholborane (5 ml, 1M /THF, 5 mmol, 2.1 éq.) a été rajouté sous agitation à TA sous argon (dégagement d'hydrogène). 3 min après, du tris(triméthylsilyl)phosphite (2.5 ml, 8.26 mmol, 3.5 éq.) a été rajouté d'un seul coup et l'agitation a été continuée pendant 16 h à TA. Du MeOH (3 ml) a été rajouté et le mélange contenant un précipité blanc a été agité pendant 30 min. Puis, un excès d'éther a été rajouté et après agitation, le précipité blanc a été séparé, lavé avec de l'éther, séché sous vide et dissout dans une solution aqueuse de HCl (2M, 4 ml) à TA. Après 10 min, la solution a été basifiée jusqu'à pH=8-9 avec du NaOH concentré et la solution obtenue a été chromatographiée (C18, gradient de 3% MeOH/H₂O à 50% MeOH/H₂O). Ainsi, 435 mg de **20** a été obtenu avec un rendement de 40%. La molécule a été caractérisée par son spectre RMN 1H et son spectre de masse (ES).

Par des synthèses similaires, une série d'analogues de la molécule **17** a été synthétisée. Cette série comporte des exemples de noyaux aryle/hétéroaryle différents, de groupements protecteurs alternatifs ainsi que des aldéhydes libres :

Les composés ci-dessus ont été préparés selon un protocole de synthèse analogue à celui du composé 17. Pour les composés 9e3 et 9e4, les fonctions aldéhydes ont été protégées selon les protocoles qui suivent :

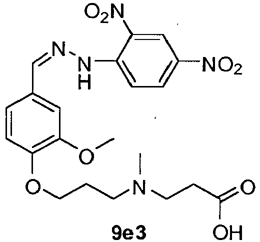

Composé 9e3 : Du 2,4-dinitrophénylhydrazine (447 mg, 1,356 mmol, 2 éq.) a été rajouté à la solution de 9e1 (200 mg, 0,678 mg, 1 éq.) dans MeOH (5 ml) et la suspension obtenue a été agitée à TA pendant 3h. Le précipité rouge a été filtré et introduit sur une colonne de gel de silice dans une solution de DMSO. Après élution (gradient de MeOH à l'eau), 140 mg (43%) de produit final 9e3 ont été obtenus sous forme d'une poudre rouge foncé.

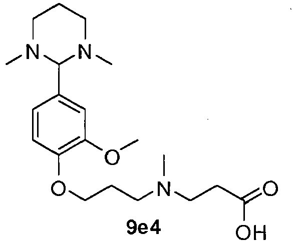

Composé 9e4 : Une solution de 9e1 (100 mg, 0,339 mg, 1 éq.) et de N,N'-diméthyl-(1,3-diaminopropane) (35 mg, 0,34 mmol, 1 éq.) dans du MeOH (1 ml) a été évaporée à sec pendant 15 min à 50°C pour obtenir le produit final pur 9e4 (126 mg, 98 %).

Tous ces produits ont été caractérisés par leurs spectres RMN 1H.

### 1.2. Synthèse des molécules de formule (I)

- Le composé **3** selon l'invention et le composé **4** de référence, dérivés de podophyllotoxine, ont été préparés à partir du composé **20** précédent selon le schéma général suivant :

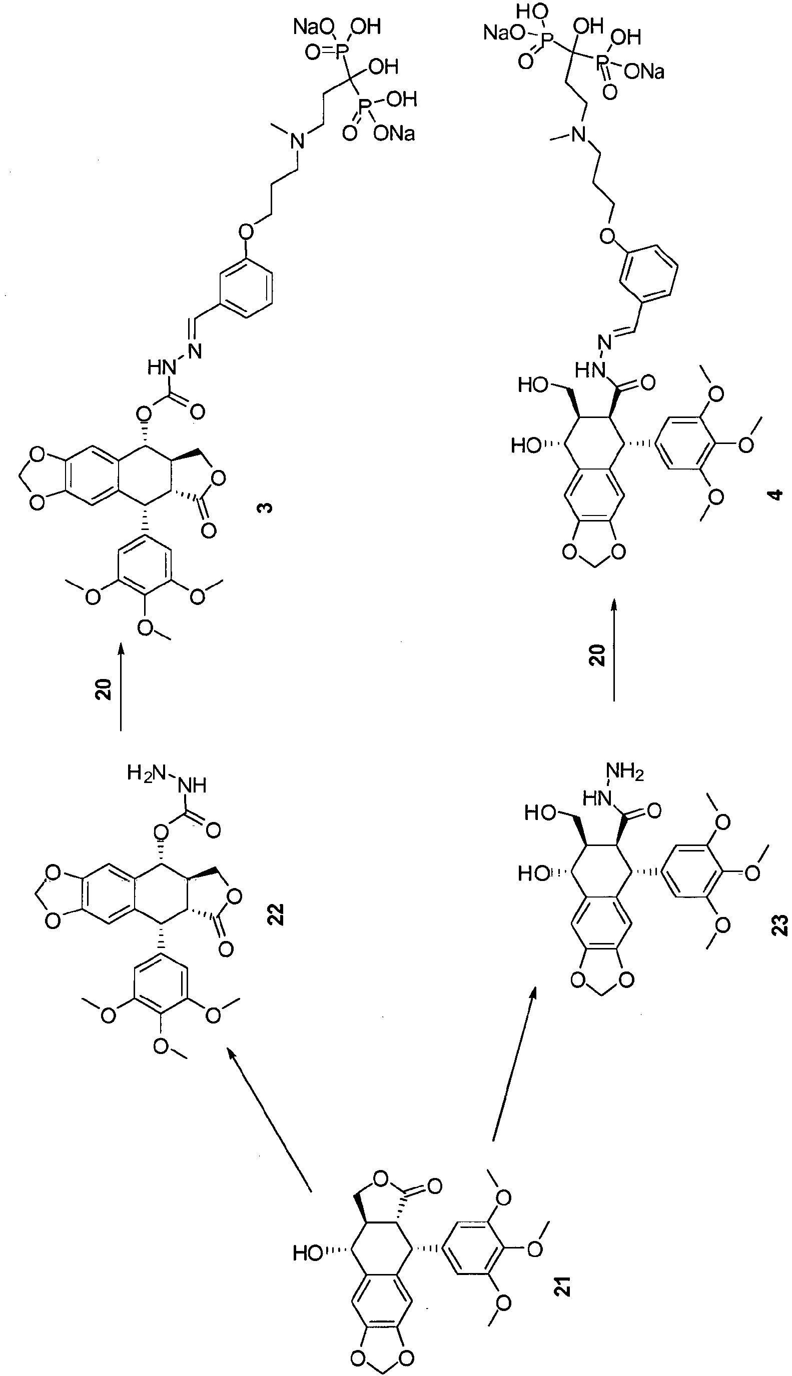

### Composé 22 :

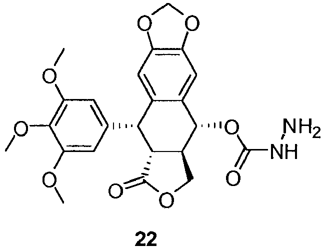

Du DCM (6 ml) a été rajouté à un mélange sec de podophyllotoxine **21** (90 mg, 0.217 mmol, 1 éq.) et de CDI (176 mg, 1.085 mmol, 5 éq.) à TA sous argon. Après 1 h, NH₂NH₂·H₂O (1M/MeCN, 2.17 ml, 2.17 mmol, 10 éq.) a été rajouté à la solution transparente obtenue. Après 10 min, le mélange réactionnel a été introduit sur une colonne de gel de silice (60 cm³) et chromatographié (gradient de DCM à DCM :MeOH=10 :1) pour donner le composé **22** sous forme de poudre blanche (99 mg, rendement = 97%). La molécule a été caractérisée par son spectre RMN 1H.

### Composé 23 :

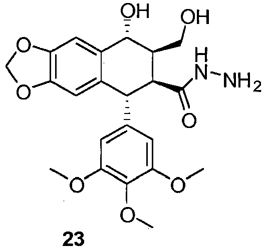

NH₂NH₂·H₂O (2 ml) a été rajouté à une solution de podophyllotoxine (400 mg, 0.966 mmol) dans du MeOH (8 ml). Cette solution a été concentrée sous vide à 50 °C et le résidu obtenu a été chromatographié sur gel de silice (gradient de DCM à MeOH). Ainsi, le composé **23** a été obtenu sous forme d'une mousse blanche solide (410 mg, rendement = 89%). La molécule a été caractérisée par son spectre RMN 1H.

### Composé 3 :

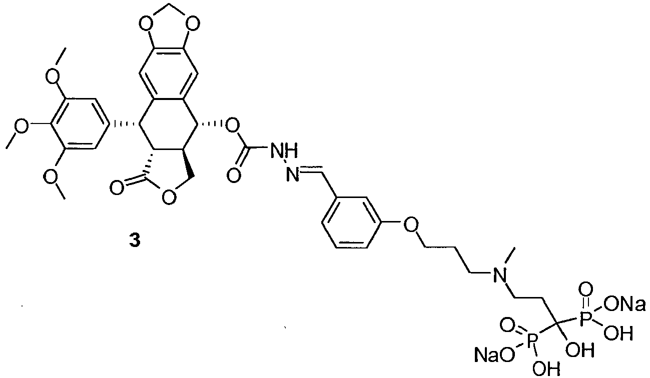

Une solution de **20** (96 mg, 0.21 mmol, 1 éq.) dans l'eau (1.5 ml) a été rajoutée à une solution de **22** (119 mg, 0.252 mmol, 1.2 éq.) dans du MeOH (1.5 ml). Le mélange obtenu a été laissé à TA pendant 16 h. Après dilution avec du iPrOH, le précipité formé a été filtré, lavé avec du iPrOH et séché. 150 mg du composé **3** brut ont été obtenus avec un rendement de 80%. Après purification chromatographique (C18, gradient de 3% MeOH/H₂O à MeOH), évaporation et cristallisation (H₂O-iPrOH-Et₂O), 75 mg du composé **3** ont été obtenus sous forme d'une poudre blanche (rendement = 40%). La molécule a été caractérisée par son spectre RMN 1H et son spectre de masse (ES).

### Composé 4 :

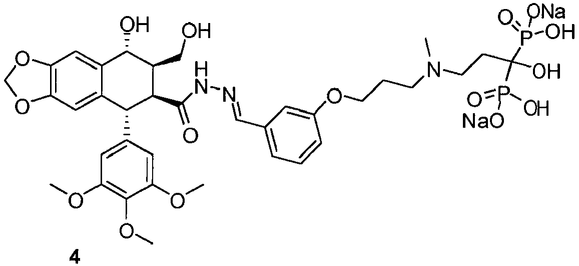

A une suspension de **23** (94 mg, 0.21 mmol, 1 éq.) dans l'eau (1 ml), du NaOH (2M, 5 gouttes) a été rajouté. A la solution obtenue légèrement trouble, une solution de **20** (136 mg, 0.3 mmol, 1.4 éq.) dans l'eau (1 ml) a été rajoutée et le mélange obtenu a été bien agité. Après 1 h, le pH de la solution obtenue légèrement trouble a été réglé à 7 avec quelques gouttes de tampon phosphate concentré. Après 16 h à TA, la solution presque transparente a été introduite sur une colonne C18. Après élution (gradient de 3% MeOH/H₂O à 50% MeOH/H₂O), le composé **4** a été obtenu (150 mg, rendement = 81%). La molécule a été caractérisée par son spectre RMN 1H et son spectre de masse (ES).
- Le composé 32 de référence, analogue azoté du gaz moutarde (agent antitumoral alkylant), a été préparé selon le schéma réactionnel suivant :

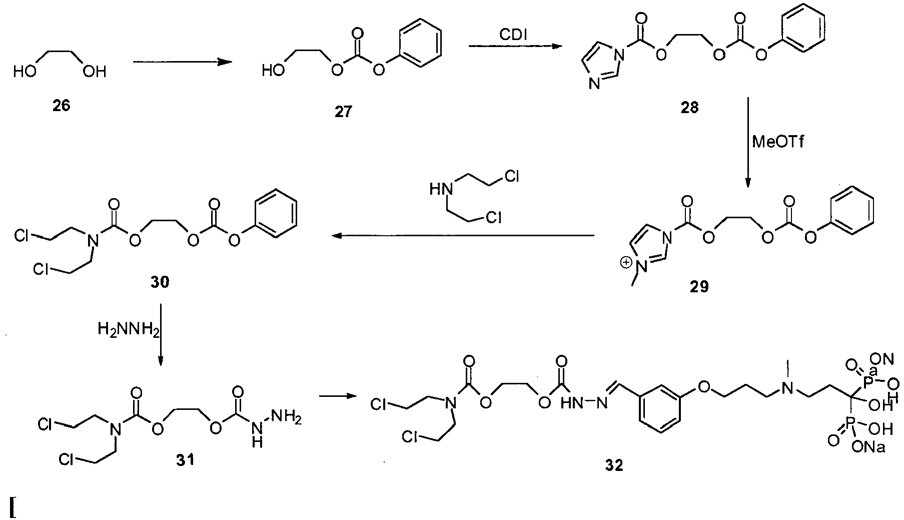

### Composé 27 :

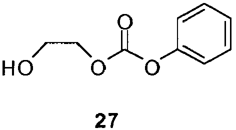

Du THF (20 ml) et du DCM (10 ml) ont été rajoutés à du NaHCO₃ (3 g) sous argon, puis de l'éthylène glycol (8 ml, 144 mmol, 5.8 éq.) suivi de chloroformiate de phényle (3.2 ml, 25 mmol, 1 éq.) à TA. Le mélange réactionnel a été bien agité pendant 5 h à TA (dégagement de CO₂ observé). Du DCM a ensuite été rajouté (150 ml) et le mélange obtenu a été bien agité. La solution a été filtrée à travers du Na₂SO₄ anhydre et le résidu obtenu a été lavé plusieurs fois avec du DCM filtré à travers du Na₂SO₄ anhydre. La solution obtenue a été concentrée sous vide et chromatographiée sur gel de silice (gradient de DCM à Et₂O) ce qui a donné le composé **27** sous forme d'une huile incolore (2.5 g, rendement = 56%). La molécule a été caractérisée par son spectre RMN 1H.

### Composé 28 :

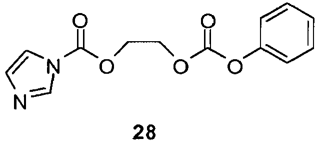

Du CDI (1.39 g, 8.58 mmol, 3 éq.) a été rajouté à une solution de **27** (520 mg, 2.86 mmol, 1 éq.) dans du THF (4 ml) à TA sous argon. Après 10 min, le précipité formé a été séparé et la solution restante a été introduite sur une colonne de gel de silice et chromatographiée (Et₂O) pour donner le composé **28** (270 mg, rendement = 34%). La molécule a été caractérisée par son spectre RMN 1H.

### Composé 30 :

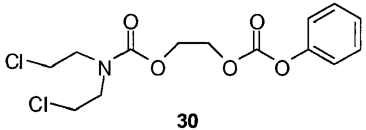

Du MeOTf (97 mg, 67 µl, 0.59 mmol, 1.2 éq.) a été rajouté à une solution de **28** (135 mg, 0.49 mmol, 1 éq.) dans du DCM (3 ml) à 0°C sous argon. Après 1 h d'agitation, du di(2-chloroéthyl)amine (139 mg, 124 µl, 0.98 mmol, 2 éq.), fraîchement préparé à partir de son chlorhydrate (extraction DCM/2M NaHCO₃, la phase organique ayant été séchée sur du Na₂SO₄ anhydre et concentrée sous vide à TA), a été rajouté et le mélange réactionnel a été laissé se réchauffer progressivement à TA pendant 3 h. Le mélange réactionnel résultant a ensuite été introduit sur une colonne de gel de silice et chromatographié (gradient du cyclohexane à Et₂O) pour donner le composé **30** (130 mg, rendement = 76%). La molécule a été caractérisée par son spectre RMN 1H.

### Composé 31 :

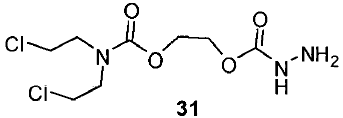

Du NH₂NH₂·H₂O (1M/MeCN, 342 µl, 342 mmol, 2 éq.) a été rajouté à une solution de **30** (60 mg, 0.171 mmol, 1 éq.) dans du DCM (1 ml). Après 40 min, le mélange réactionnel a été introduit sur une colonne de gel de silice (20 cm³) et chromatographié (gradient de DCM à DCM :MeOH=10 :1) pour donner le composé **31** sous forme d'une huile jaune clair (32 mg, 65%). La molécule a été caractérisée par son spectre RMN 1H.

### Composé 32 :

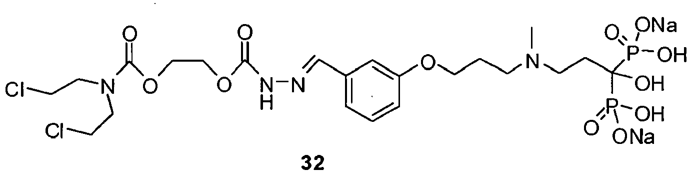

Une solution de **20** (46 mg, 0.101 mmol, 1 éq.) dans de l'eau (300 µl) a été rajoutée à une solution de **31** (29 mg, 0.101 mmol, 1 éq.) dans du MeOH (300 µl) à TA. Une huile se sépare alors du mélange trouble obtenu. Après agitation, l'huile se transforme progressivement en cristaux. Le mélange a été laissé à TA pendant 16 h, puis les cristaux ont été filtrés, lavés (50% MeOH dans l'eau, puis avec MeOH) et séchés sous vide. Ainsi, le composé **32** a été obtenu (30 mg, rendement = 41%). La molécule a été caractérisée par son spectre RMN 1H et son spectre de masse (ES).
- Le composé **36** selon l'invention, analogue du composé **3**, a été préparé selon le schéma réactionnel suivant, selon des protocoles décrits précédemment : 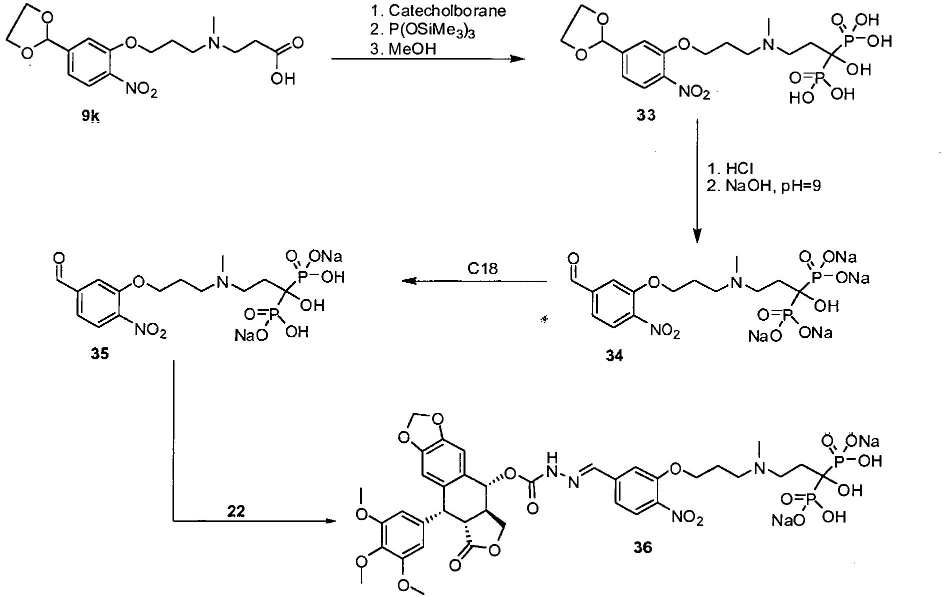 La molécule **36** a été caractérisée par ses spectres RMN 1H et 31P.
- Les molécules **41** et **42**, composés selon l'invention, portant un résidu de gaz moutarde, ont été synthétisées selon le schéma réactionnel suivant, selon des protocoles décrits précédemment : 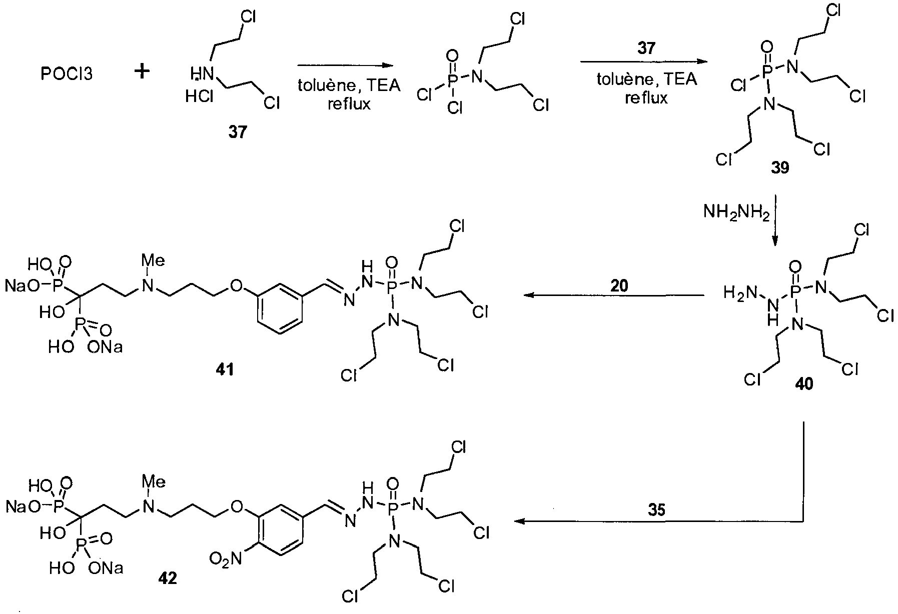 Les molécules **41** et **42** ont été caractérisées par leurs spectres RMN 1H et 31P.
- Le composé **46** selon l'invention, portant un résidu de gaz moutarde, a été synthétisé selon le schéma réactionnel suivant, selon des protocoles décrits précédemment : 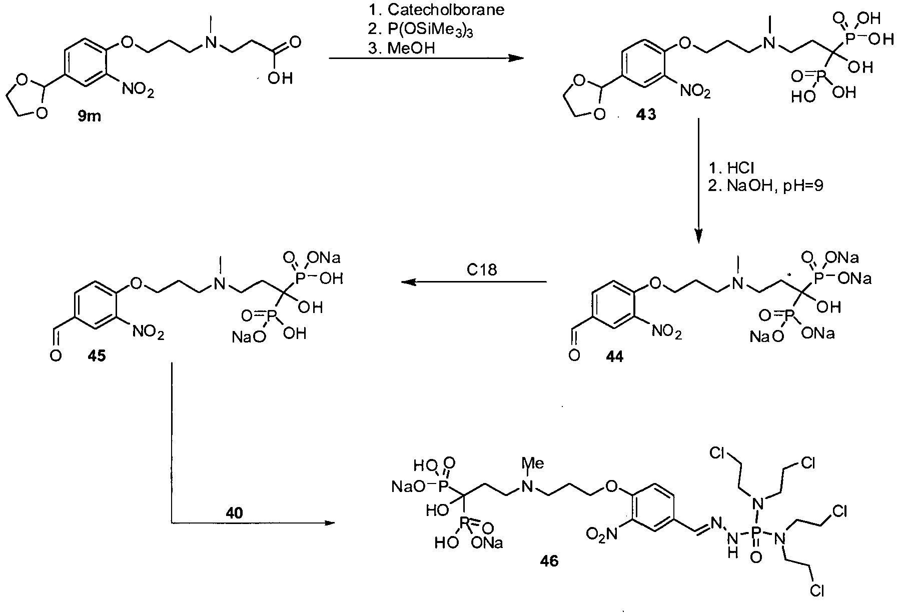 La molécule **46** a été caractérisée par son spectre RMN 1H et 31P.
- La molécule **48** de référence, portant un résidu de dansyle utile pour l'imagerie médicale par fluorescence, a été synthétisée selon le schéma réactionnel suivant à partir du composé **47** commercial, selon des protocoles décrits précédemment : 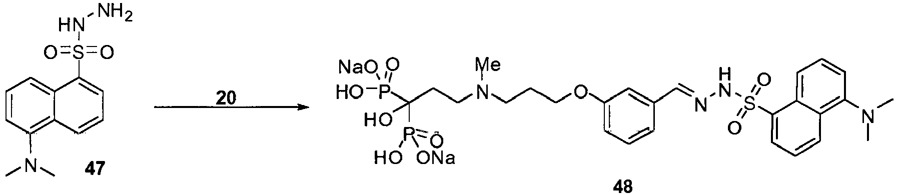 La molécule **48** a été caractérisée par son spectre RMN 1H et 31P.
- La molécule **50** de référence, portant un résidu de fluorescéine modifiée utile pour l'imagerie médicale par fluorescence, a été synthétisée selon le schéma réactionnel 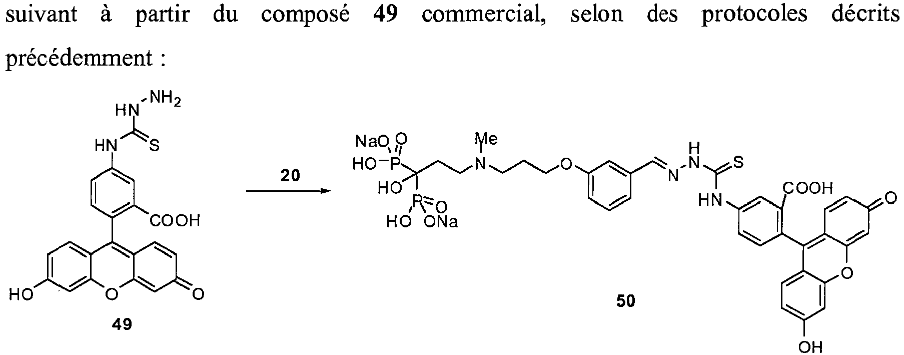 La molécule **50** a été caractérisée par son spectre RMN 1H et 31P.
- Les molécules **55** et **56** de référence, portant un résidu de pyrène utile pour l'imagerie médicale par fluorescence, a été synthétisée selon le schéma réactionnel suivant :

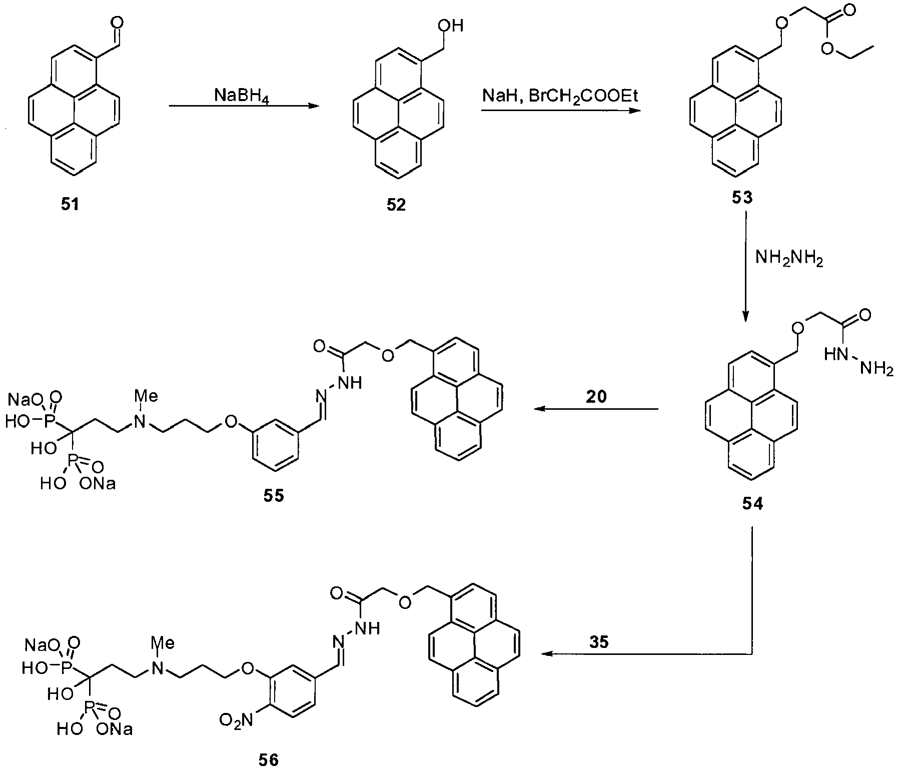

### Composé 52 :

NaBH₄ (124 mg, 3.27 mmol) a été rajouté à une solution du composé **51** commercial (250 mg, 1.09 mmol) dans MeOH (2 ml). 5min après, le pH a été ajusté à 1 avec une solution de HCl 1M et le mélange obtenu a été dilué avec de l'eau. Le précipité a été filtré, lavé avec de l'eau, et séché. 250 mg du composé **52** ont ainsi été obtenus avec un rendement de 99%.

### Composé 54 :

NaH (41 mg, 1.03 mmol) a été rajouté à une solution du composé **52** (200 mg, 0.862 mmol) dans le diméthylformamide (3 ml) et le mélange a été agité 2 min à température ambiante. De l'éthylbromoacétate (144 µl, 1.03 mmol) a été rajouté à la même température et l'agitation a été continuée pendant 1 h. NH₂NH₂·H₂O (1 ml) a été rajouté et l'agitation a été continuée pendant 2 h à température ambiante. De l'eau (40 ml) a été rajoutée suivie de cyclohexane (40 ml). Le précipité obtenu a été filtré, lavé avec du MeOH, et séché. 102 mg du composé **54** ont ainsi été obtenus avec un rendement de 39% (sur deux étapes).

### Composés 55 et 56 :

Ces composés ont été obtenus à partir du composé 54 par des protocoles décrits précédemment.
Les molécules **55** et **56** ont été caractérisées par les spectres RMN 1H et 31P.
- La molécule **57** de référence, portant un résidu de fluorescéine modifiée utile pour l'imagerie médicale par fluorescence, a été synthétisée selon le schéma réactionnel suivant à partir du composé **49** commercial, selon des protocoles décrits précédemment : 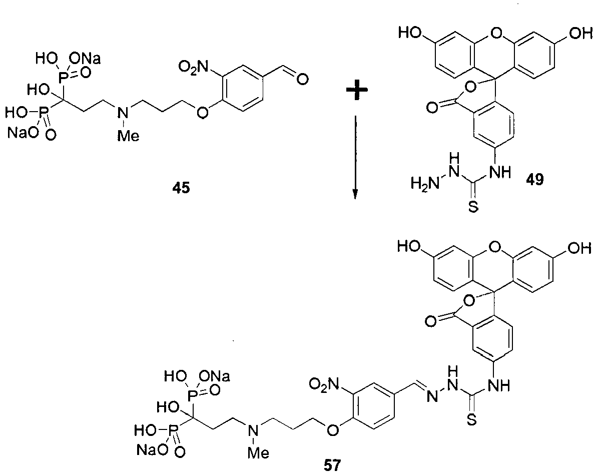 La molécule **57** a été caractérisée par son spectre RMN 1H et 31P.
- La molécule **58** de référence, portant un résidu de dansyle utile pour l'imagerie médicale par fluorescence, a été synthétisée selon le schéma réactionnel suivant à partir du composé **47** commercial, selon des protocoles décrits précédemment : 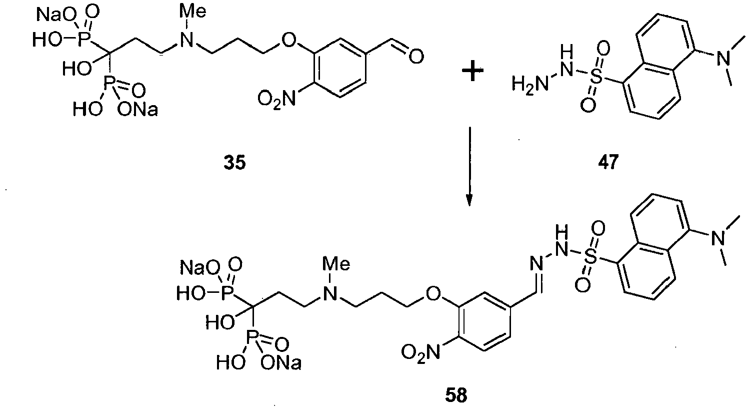 La molécule **58** a été caractérisée par son spectre RMN 1H et 31P.
- La molécule **59** de référence, portant un résidu de rhodamine B utile pour l'imagerie médicale par fluorescence, a été synthétisée selon le schéma réactionnel suivant :

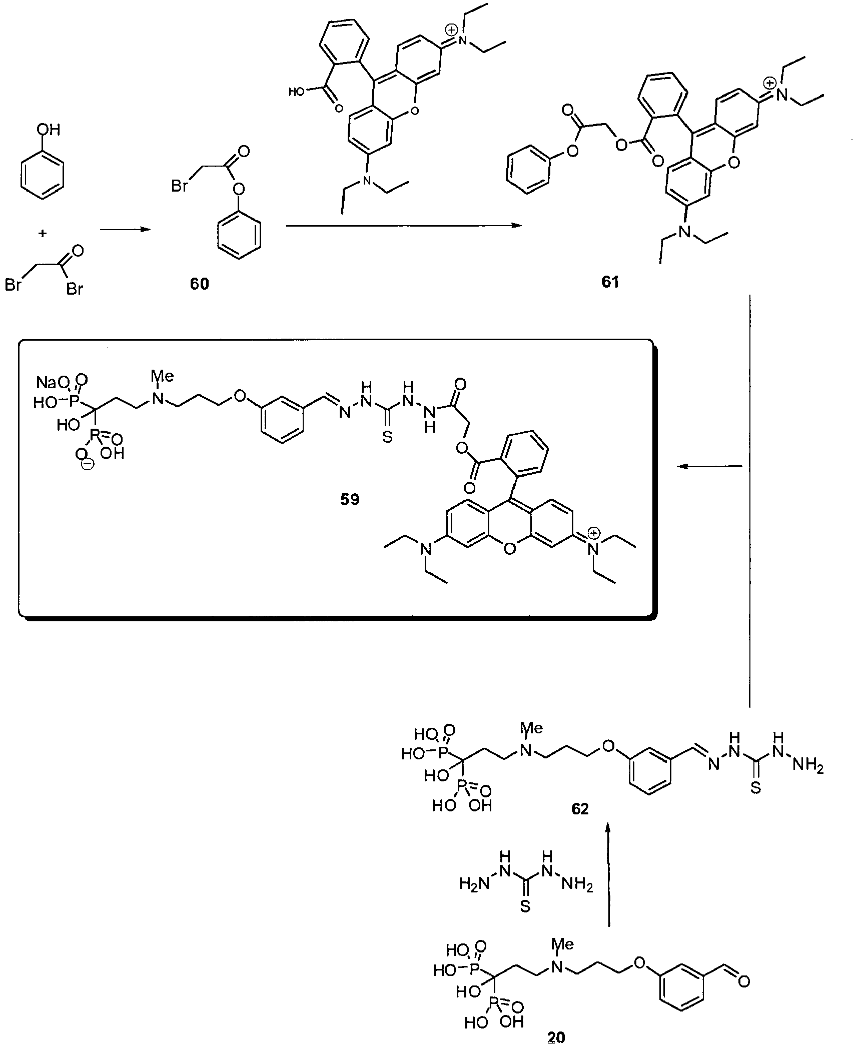

### Composé 60 :

Le mélange de phénol (10 g, 106 mmol) et de bromoanhydride de l'acide bromoacétique (9.16 ml, 106 mmol) a été chauffé à 90°C pendant 10 min, puis tous les composants volatiles ont été évaporés à sec à 90°C sous vide. 23 g du composé **60** ont ainsi été obtenus avec un rendement quasiment quantitatif.

### Composé 61 :

Une solution du composé **60** (0.243 ml, 2.2 mmol) et de rhodamine B commerciale (884 mg, 2 mmol) dans MeNO₂ (5 ml) a été agitée à 80°C pendant 5 min, puis évaporée à sec sous vide à la même température. Le composé **61** a ainsi été obtenu sous forme de solide noire amorphe (1.22 g, rendement≅100%).

### Composé 62 :

Une solution de thiocarbohydrazide (233 mg, 2.2 mmol) dans NaOH 2M (2 ml) a été rajoutée à une solution de **20** (100 mg, 0.22 mmol) dans l'eau (2 ml). La solution transparente a été évaporée à 60°C à sec. De l'eau (2 ml) a été rajoutée et la solution a été reévaporée à 60°C à sec. Le résidu a été chromatographié sur colonne C18 pour donner le composé **62**, 71 mg, solide amorphe, rendement = 60%.

### Composé 59 :

Une solution de **62** (10 mg, 0.0184 mmol) et de **61** (48 mg, 0.073 mmol) dans un mélange d'eau (1 ml) et de THF (1 ml) a été agitée 2 h à 40°C. Du MeOH suivi de Et₂O ont ensuite été rajoutés. Le précipité formé a été filtré (14 mg) et chromatographié sur colonne C18. 2 mg du composé **59** ont ainsi été obtenus, rendement = 10%.
La molécule **59** a été caractérisée par son spectre RMN 1H et 31P.
- La molécule **63** de référence, portant un résidu de gaz moutarde, a été synthétisée selon le schéma réactionnel suivant :

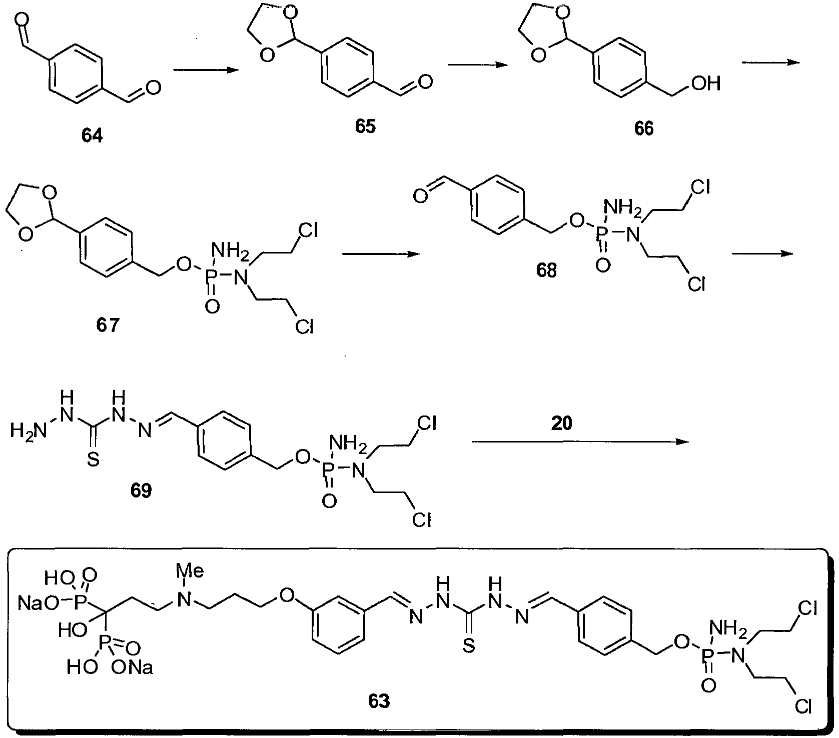

### Composé 65 :

Un mélange de **64** (10 g, 74.6 mmol), de glycérol (4.6 g, 74.6 mmol) et d'acide *para-*toluènesulfonique (710 mg, 3.7 mmol) dans du benzène a été chauffé à reflux (Dean-Stark) pendant 5 h, puis refroidi à la TA. Un excès de K₂CO₃ anhydre a été rajouté, le mélange a été bien agité, puis filtré à travers K₂CO₃ anhydre, évaporé sous vide, chromatographié sur gel de silice. Le composé **65** a ainsi été obtenu (6.3 g, rendement=47%) sous forme d'huile légèrement jaune.

### Composé 66 :

Du NaBH₄ (427 mg, 11.2 mmol) a été rajouté progressivement à une solution refroidie avec de l'eau froide de **65** (1 g, 5.6 mmol) dans MeOH (5 ml) et le mélange obtenu a été agité 5 min à la TA. Le pH du mélange a été ajusté à 7 avec un tampon phosphate. Un mélange MeOH : DCM=1:1 a été rajouté suivi de Na₂SO₄ anhydre et le mélange final a été bien agité, puis filtré à travers Na₂SO₄ anhydre et concentré sous vide. Le composé **66** a été obtenu (980 mg, rendement=96%).

### Composé 67 :

Du HMDS-Na (2M dans le THF, 1.67 ml, 3.34 mmol) est rajouté à -120°C sous l'argon à une solution de **66** (500 mg, 2.78 mmol) dans le THF (23 ml). Après 10 min à -120°C, du dichlorure bis(2-chloroéthyl)phosphoramidique (720 mg, 2.78 mmol) dans le THF (4 ml) a été rajouté d'un seul coup. Le mélange réactionnel est réchauffé progressivement à la TA pendant 1.5 h, puis refroidi à -90°C. A cette température, une solution d'ammoniac dans le DCM (1.5 M, 5.7 ml, 8.62 mmol) y a été rajoutée. Le bain froid a été enlevé, le mélange réactionnel a été agité à la TA pendant 1 h, de la silice avec du MeOH a été rajouté et le solvant a été évaporé sous pression réduite à 40°C. Après chromatographie sur silice (gradient à partir de DCM jusqu'au DCM : MeOH = 5 : 1) et évaporation du solvant, le composé **67** est obtenu (1.03 mg, 96%).

### Composé 68 :

Le composé **67** (1 g, 2.6 mmol) a été solubilisé dans 15 ml de DCM où 50 ml de Et₂O a été rajouté. La solution obtenue a été bien secouée avec du HCl 2M, lavée avec de l'eau, puis avec une solution saturée de NaHCO₃. La phase organique a été séchée avec Na₂SO₄ anhydre et concentrée sous vide à 40°C. Le composé **68** est obtenu (610 mg, 69%).

### Composé 69 :

Une solution de thiocarbohydrazide (400 mg, 3.78 mmol) dans du NaOH 1M (6 ml) a été rajoutée à une solution de **68** (128 mg, 0.378 mmol) dans MeOH (11 ml) à la TA. Après 40 min le mélange a été dilué avec une solution saturée de NaCl et extrait avec DCM (5 fois). La phase organique a été séchée avec Na₂SO₄ anhydre et concentrée sous vide à 40°C. Le composé **69** est obtenu (50 mg, 31%).

### Composé 63 :

Une solution de **20** (50 mg, 0.11 mmol) dans de l'eau (1 ml) a été rajoutée à une solution de **69** (50 mg, 0.117 mmol) dans le THF (3 ml) à la TA. La solution obtenue a été évaporée sous vide à 60°C. Du THF (3 ml) suivi d'eau (0.5 ml) ont été rajoutés au résidu obtenu et le mélange a été chauffé 5 min à 60°C. 3 gouttes de NaOH 2M ont été rajoutées à la solution obtenue et le mélange réactionnel (pH=9-10) a été laissé pour 16 h à la TA. Après une purification par chromatographie sur colonne C18, le composé **63** (20 mg, rendement=21%) a été obtenu.
La molécule **63** a été caractérisée par son spectre RMN 1H et 31P.
- La molécule **70** de référence, portant un résidu d'ibuprofène, a été synthétisée selon le schéma réactionnel suivant :

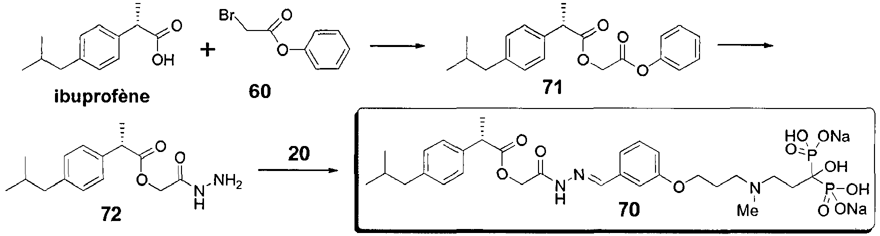

### Composé 72 :

A la TA, le composé **60** (312 mg, 1.45 mmol) a été rajouté à un mélange d'ibuprofène (250 mg, 1.21 mmol) et de Na₂CO₃ (128 mg, 1.21 mmol) dans du DMF (3 ml) et le mélange obtenu a été agité 30 min à la TA. A ce mélange, contenant le composé **71** brut, du DCM (20 ml) a été rajouté à la TA, suivi d'hydrate d'hydrazine (1M dans MeCN, 3.7 ml, 3.7 mmol), le mélange obtenu a été agité 30 min, puis dilué avec de l'éther (60 ml), extrait 5 fois avec du NaOH 0.1M, séché avec du Na₂SO₄ anhydre et concentré sous vide à 40°C. Le composé **72** (170 mg, rendement=51% sur deux étapes) a été obtenu,

### Composé 70 :

Une solution de **20** (25 mg, 0.0568 mmol) et de **72** (19 mg, 0.0681 mmol) dans un mélange H₂O:THF=1:1 (3 ml) a été évaporé sous vide à 60°C. Le même cycle solubilisation/évaporation a été répété 2 fois. Après une purification par chromatographie sur colonne C18, le composé **70** (24 mg, rendement=49%) a été obtenu.

La molécule **70** a été caractérisée par son spectre RMN 1H et 31P.
- La molécule **73** selon l'invention, portant un résidu de produit anticancéreux méthotrexate, a été synthétisée selon le schéma réactionnel suivant :

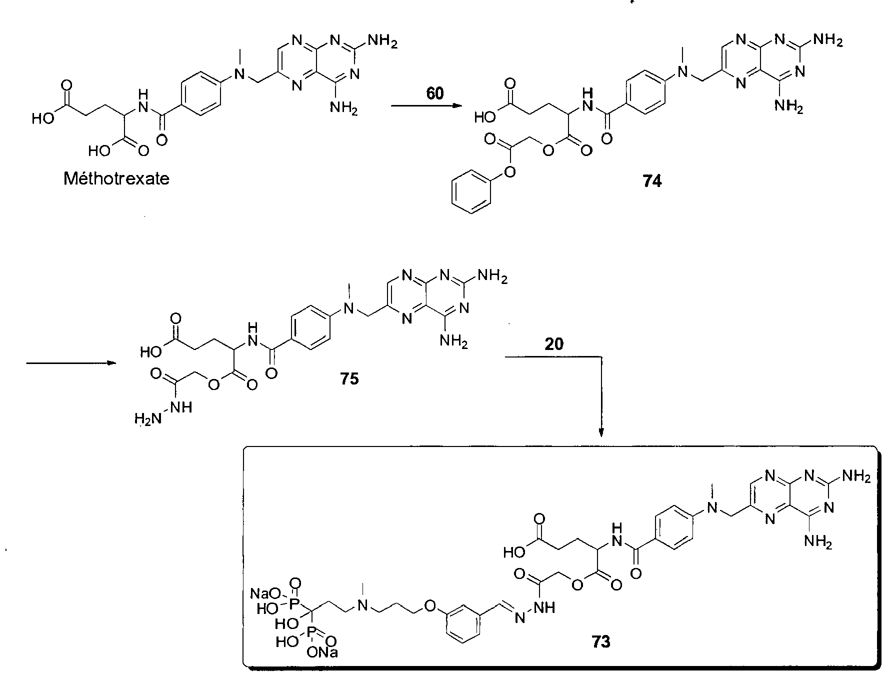

### Composé 74 :

A la TA, le composé **60** (57 mg, 0.264 mmol) a été rajouté à un mélange de méthotrexate (100 mg, 0.22 mmol) et de Na₂CO₃ (23 mg, 0.22 mmol) dans du DMF (1 ml) et le mélange obtenu a été agité 24 h à la TA. Ce mélange a été chromatographié sur silice (DCM→MeOH). Le composé **74** (45 mg, rendement=35%) a été obtenu.

### Composé 75 :

Un mélange de **74** (40 mg, 0.0679 mmol), MeOH (16 ml), DCM (10 ml), THF (2 ml) et hydrate d'hydrazine (1M dans MeCN, 0.1 ml, 0.1 mmol) a été évaporé à 40°C sous vide. Le résidu a été solubilisé dans 1 ml d'eau, dilué avec MeOH, puis avec un excès de Et₂O. Le précipité obtenu a été filtré, lavé avec Et₂O et séché. Le composé **75** (30 mg, rendement=84%) a été obtenu.

### Composé 73 :

Une solution de **20** (32 mg, 0.0712 mmol) et de **75** (25 mg, 0.0474 mmol) dans un mélange H₂O:THF=1:1 (3 ml) a été évaporée sous vide à 60°C. Le même cycle solubilisation/évaporation a été répété 2 fois. Après une purification par chromatographie sur colonne C18, le composé **73** (23 mg, rendement=50%) a été obtenu.
La molécule **73** a été caractérisée par son spectre RMN 1H et 31P.
- La molécule **80** selon l'invention, portant un résidu de produit anticancéreux méthotrexate, a été synthétisée selon le schéma réactionnel suivant :

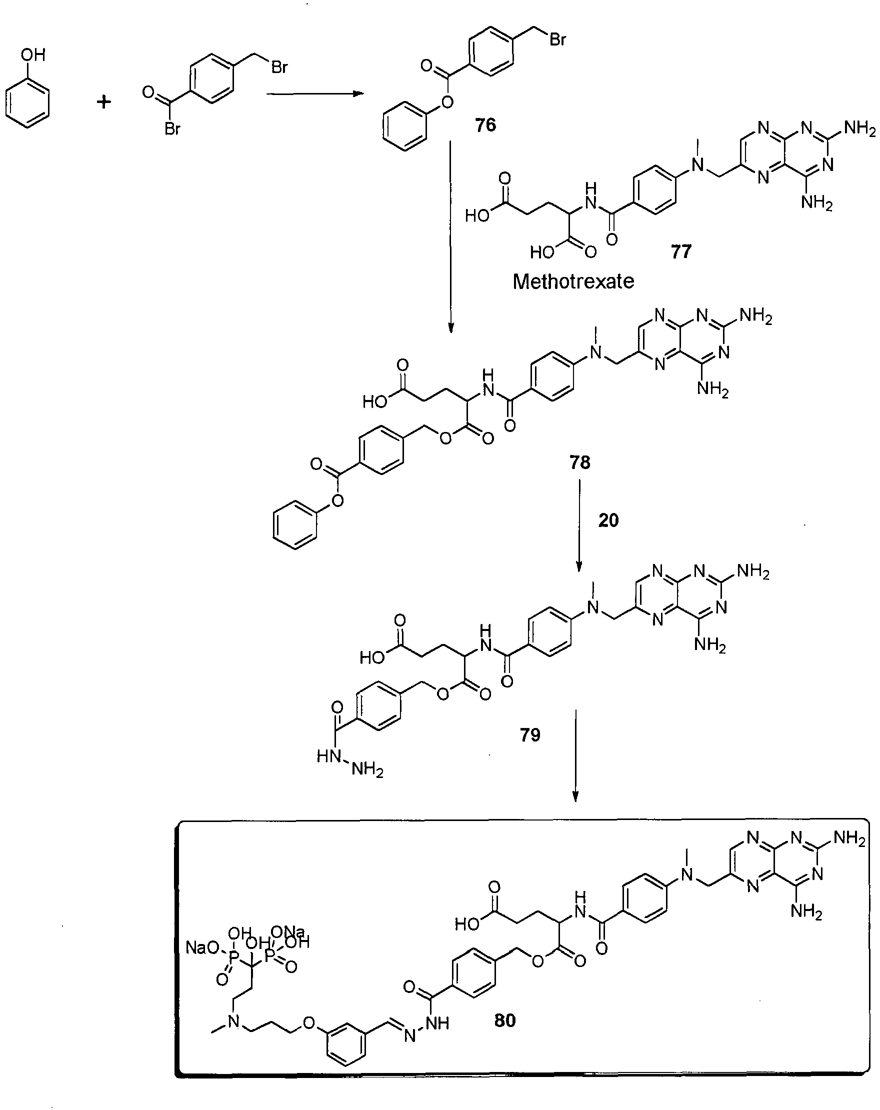

### Composé 76 :

Il a été préparé de la même façon que le composé **60.** Le produit final cristallin a été obtenu avec un rendement quasiment quantitatif.

### Composé 78 :

Il a été préparé de la même façon que le composé **74** en partant des composés **76** et **77**, avec un rendement de 38%. La molécule **78** a été caractérisée par son spectre RMN 1H.

### Composé 79 :

Il a été préparé de la même façon que le composé **75** en partant du composé **78,** avec un rendement de 77%. La molécule **79** a été caractérisée par son spectre RMN 1H.

### Composé 80 selon l'invention:

Il a été préparé de la même façon que le composé **73** en partant des composés **79** et **20,** avec un rendement de 65%. La molécule **80** a été caractérisée par son spectre de masse, RMN 1H et 31P.
- La molécule **90** selon l'invention, portant un résidu de produit anticancéreux méthotrexate, a été synthétisée selon le schéma réactionnel suivant :

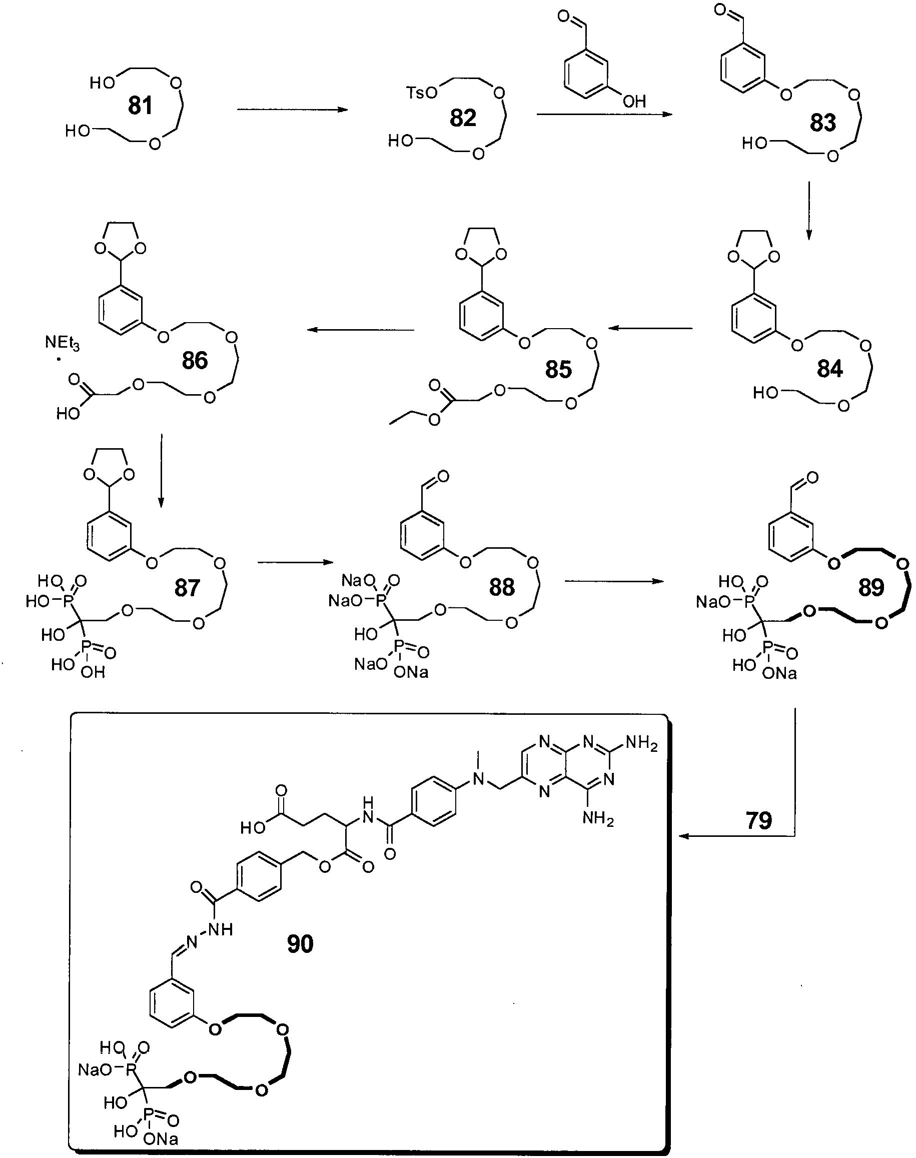

### Composé 83 :

Du chlorure de tosyle TsCl (1.01 g, 5.3 mmol) a été ajouté au mélange du composé **81** (4.7 ml, 6 éq.) et de K₂CO₃ (814 mg, 5.9 mmol) agité 3 min à 80 °C et le mélange obtenu a été agité 5 min de plus. Puis le mélange sec de K₂CO₃ (814 mg, 5.9 mmol), de 3-hydroxybenzaldéhyde (500 mg, 4.1 mmol) et de NaI (73 mg) a été rajouté et le mélange obtenu a été agité 16 h à 80 °C. Une solution 2M de NaOH (1 ml) a été rajoutée et le mélange a été agité 10 min à température ambiante. Après une extraction avec DCM-H₂O-NaCl-NaOH, la solution organique a été séchée avec Na₂SO₄ et concentrée sous vide. Le composé **83** pur a été obtenu (800 mg) avec un rendement de 77%. La molécule **83** a été caractérisée par son spectre RMN 1H.

### Composé 84 :

Du Na₂SO₄ anhydre (2.5 g) a été rajouté à une solution du composé **83** (800 mg, 3.15 mmol) dans du benzène (10 ml). A cette solution ont été rajoutés de la pyridine (12 µl, 0.158 mmol), du PTSA·H₂O (30 mg, 0.158 mmol) et de l'éthylène glycol (578 µl, 10.4 mmol) et le mélange obtenu a été agité à 77 °C pendant 3 h. Du K₂CO₃ (1 g) a été rajouté et après 15 min d'agitation et refroidissement, le mélange a été filtré à travers K₂CO₃ qui a été lavé avec Et₂O. Après concentration sous vide, le composé **84** pur a été obtenu (750 mg) avec un rendement de 80%. La molécule **84** a été caractérisée par son spectre RMN 1H.

### Composé 85 :

Du NaH (60% dans l'huile minérale, 403 mg, 10.08 mmol) a été rajouté à une solution du composé **84** dans le DMF (10 ml) et le mélange a été agité 5 min à température ambiante. Du bromoacétate d'éthyle (1.12 ml, 10.08 mmol) a été rajouté et le mélange a été agité 4 h à température ambiante. Du méthanol (2 ml) a été rajouté. La solution obtenue a été introduite sur une colonne de gel de silice (160 g) et éluée avec un gradient cyclohexane-DCM. La solution organique a été séchée sous vide (80°C, 40 mbar, 30 min). Le composé **85** a été obtenu (1,1 g) avec un rendement de 71%. La molécule **85** a été caractérisée par son spectre RMN 1H.

### Composé 86 :

Une solution du composé **85** (540 mg, 1.41 mmol) et de NaOH (2M, 0.6 ml) dans MeOH (2 ml) a été agitée 40 min à température ambiante, puis chromatographiée sur gel de silice (40 g, éluant : gradient de DCM à DCM : MeOH :TEA=2 :1 :0,2). La solution organique a été concentrée sous vide à 40°C puis co-évaporée 3 fois avec du THF à la même température. Le composé **86** a été obtenu (348 mg) avec un rendement de 66%. La molécule **86** a été caractérisée par son spectre RMN 1H.

### Composé 89 :

A une solution du composé **86** (60 mg, 0.14 mmol) et de TEA (5.8 µl, 0.086 mmol) dans le THF (360 µl), du catécholborane (0.3 ml, 1M /THF, 0.3 mmol) a été rajouté sous agitation, à température ambiante, sous argon (dégagement d'hydrogène). 3 min après, du tris(triméthylsilyl)phosphite (150 mg, 0.5 mmol) a été rajouté d'un seul coup et l'agitation a été poursuivie pendant 3 h à température ambiante. Du MeOH (0.4 ml) a été rajouté et le mélange a été agité pendant 15 min. Puis, un excès d'éther a été rajouté et après agitation, l'huile incolore a été séparée, lavée avec de l'éther, séchée sous vide, dissoute dans 1 ml d'eau et HCl_{conc}. (0.25 ml) à température ambiante. Après 30 min, la solution a été basifiée jusqu'à pH=14 avec du NaOH concentré, puis le pH a été ajusté à 7 avec un tampon phosphate et la solution obtenue a été chromatographiée (C18, 3% MeOH/H₂O). 15 mg de composé 86 pur ont été obtenus avec un rendement de 21%. La molécule a été caractérisée par son spectre de masse, RMN 1H et 31P.

### Composé 90 selon l'invention:

Il a été préparé de même façon que le composé **73** en partant des composés **79** et **89** et a été caractérisé.
- La molécule **94** de référence, portant un résidu de produit anticancéreux doxorubicine, a été synthétisée selon le schéma réactionnel suivant :

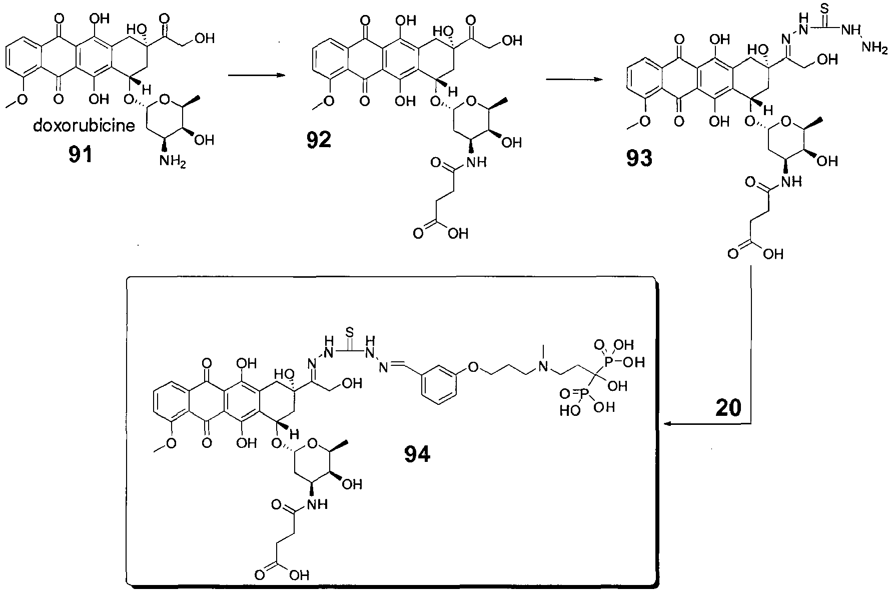

### Composé 92 :

De l'anhydride succinique (8.6 mg, 0.086 mmol) a été rajouté à un mélange de doxorubicine (50 mg, 0.086 mmol) et de TEA (0.1 ml) dans du DMF (2 ml) et le mélange obtenu a été agité 10 min à température ambiante puis chromatographié sur gel de silice (éluant : gradient de DCM à MeOH). Le composé **92** pur a été obtenu (53 mg) avec un rendement de 96%. La molécule **92** a été caractérisée par son spectre de masse et RMN 1H.

### Composé 93 :

Une solution de composé **92** (50 mg, 0.078 mmol) et de thiocarbohydrazide (55 mg, 0.52 mmol) dans un mélange MeOH : TFA 1000 : 1 a été agitée 16 h à température ambiante, puis chromatographiée sur gel de silice (éluant : gradient de DCM à DCM :MeOH=1 :1). Le composé **93** a été obtenu (53 mg) avec un rendement de 95%.

### Composé 94 :

Une solution de composé **93** (10 mg, 0.0137 mmol), de composé **20** (8 mg, 0.0176 mmol) et de 4 gouttes de AcOH dans un mélange eau :THF=1:1 (4 ml) a été agitée 16 h à température ambiante, puis le THF a été enlevé sous vide à température ambiante et le précipité formé a été filtré, lavé avec de l'eau, du MeOH et de l'Et₂O et séché. Le composé **94** a été obtenu (8 mg) avec un rendement de 52%. La molécule **94** a été caractérisée par son spectre de masse, RMN 1H et RMN 31P.
- La molécule **99** de référence, portant un résidu de produit anticancéreux SN38, a été synthétisée selon le schéma réactionnel suivant :

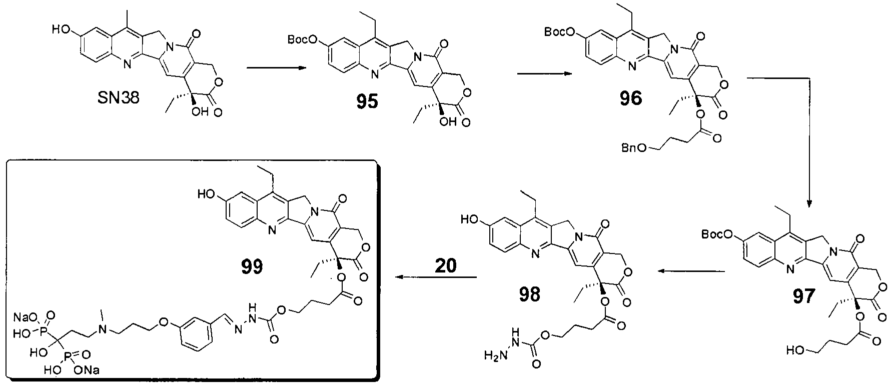

### Composé 95 :

Le composé **95** est synthétisé selon le mode opératoire décrit dans Bioconjugate Chem. 19, 4, 2008, 849-859.

### Composé 96 :

A une solution de composé **95** (400 mg, 0.81 mmol) dans le DCM (20mL) est ajouté de l'acide 4-benzyloxybutyrique (460 mg, 2.43 mmol), de la DMAP (100 mg, 0.81 mmol) et de l'EDCI (460 mg, 2.43 mmol). Le mélange obtenu est agité 2 h à température ambiante. De l'eau est ajoutée, la phase organique est séchée (Na₂SO₄) et évaporée sous vide. Le résidu est chromatographié sur gel de silice (éluant : DCM→MeOH). Le composé **96** (375mg, rendement=70%) a été obtenu. La molécule **96** a été caractérisée par son spectre RMN 1H.

### Composé 97 :

A une solution de composé **96** (375 mg, 0.56 mmol) dans un mélange dioxane/EtOH (20mL) sont ajoutés du cyclohexène (4mL) et du palladium sur charbon 10% (100mg). Le mélange obtenu est agité 24 h à 80°C, puis est filtré sur célite et évaporé sous vide. Le résidu est chromatographié sur gel de silice (éluant : DCM→MeOH). Le composé **97** (100mg, rendement=30%) a été obtenu. La molécule **97** a été caractérisée par son spectre RMN 1H.

### Composé 98 :

A une solution de composé **97** (150 mg, 0.26 mmol) dans le THF (10mL) est ajouté du CDI (210 mg, 1.29 mmol). La solution est agitée 20 minutes à température ambiante, puis de l'hydrate d'hydrazine (1M dans MeCN, 2.6 ml, 2.6 mmol) est ajouté et le milieu est agité 2h à température ambiante. Ce mélange est chromatographié sur gel de silice (éluant : DCM→MeOH). Le composé **98** (80 mg, rendement=57%) a été obtenu. La molécule **98** a été caractérisée par son spectre RMN 1H.

### Composé 99 :

Une solution de composé **20** (18mg, 0.040 mmol) et de composé **98** (24 mg, 0.044 mmol) dans un mélange H₂O:THF=1:1 (2ml) a été évaporée sous vide à 60°C. Le même cycle solubilisation/évaporation a été répété 2 fois. Après une purification par chromatographie sur colonne C18, le composé **99** (10 mg, rendement=25%) a été obtenu. La molécule **99** a été caractérisée par son spectre de masse, RMN 1H et 31P.
- La molécule **102** de référence, portant un résidu de produit anticancéreux chlorambucil, a été synthétisée selon le schéma réactionnel suivant :

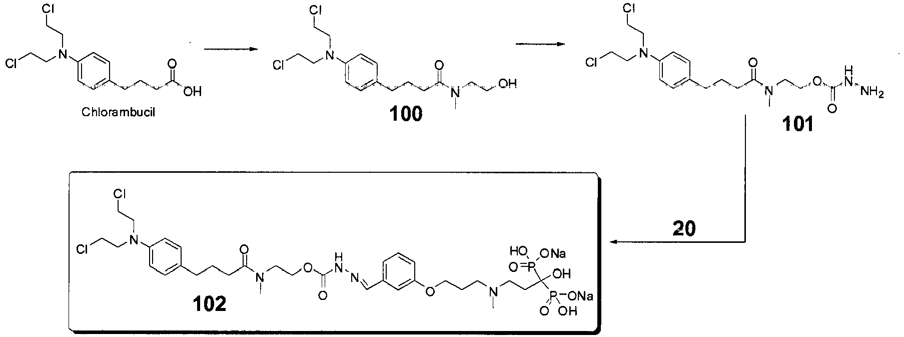

### Composé 100 :

A une solution de Chlorambucil (400 mg, 1.3 mmol) dans le DCM (20mL) sont ajoutés du 2-méthylaminoéthanol (195 mg, 2.6 mmol), de la Et₃N (0.54mL, 3.9 mmol), de l'HOBt (350 mg, 2.6 mmol) et de l'EDCI (500 mg, 2.6 mmol). Le mélange obtenu est agité 24 h à température ambiante. De l'eau est ajoutée, la phase organique est séchée (Na₂SO₄) et évaporée sous vide. Le résidu est chromatographié sur gel de silice (éluant : DCM→MeOH). Le composé **100** (375mg, rendement=80%) a été obtenu. La molécule **100** a été caractérisée par son spectre RMN 1H.

### Composé 101 :

A une solution de composé **100** (200 mg, 0.55 mmol) dans le THF (10mL) est ajouté du CDI (448 mg, 2.77 mmol). La solution est agitée 20 minutes à température ambiante, puis de l'hydrate d'hydrazine (1M dans MeCN, 3.8 ml, 3.8 mmol) est ajouté et le milieu est agité 2h à température ambiante. Ce mélange est chromatographié sur gel de silice (éluant : DCM→MeOH). Le composé **101** (100 mg, rendement=43%) a été obtenu. La molécule **101** a été caractérisée par son spectre RMN 1H.

### Composé 102 :

Une solution de composé **20** (27 mg, 0.059 mmol) et de composé **101** (30 mg, 0.071 mmol) dans un mélange H₂O:THF=1:1 (2ml) a été évaporée sous vide à 60°C. Le même cycle solubilisation/évaporation a été répété 2 fois. Après une purification par chromatographie sur colonne C18, le composé **102** (13 mg, rendement=25%) a été obtenu. La molécule **102** a été caractérisée par son spectre de masse, RMN 1H et 31P.
- La molécule **104** de référence, portant un résidu de produit hormonal estrone, a été synthétisée selon le schéma réactionnel suivant :

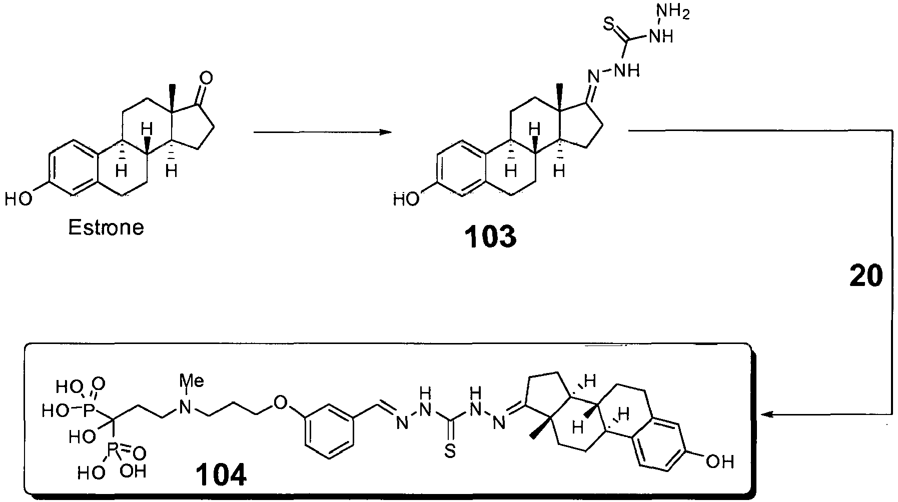

### Composé 103 :

Un mélange d'estrone (50 mg, 0.183 mmol) et de thiocarbohydrazide (97 mg, 0.916 mmol) a été solubilisé dans un mélange de NaOH 2M (1 ml) et de MeOH (1 ml) à 90°C et agité 5 min a cette température, puis refroidi et chromatographié sur gel de silice (éluant : gradient de DCM à MeOH). Le composé **103** pur a été obtenu (60 mg, rendement=92%). La molécule **103** a été caractérisée par son spectre de masse et RMN 1H.

### Composé 104 :

Un mélange de composé **103** (55 mg, 0.153 mmol) et de composé **20** (80 mg, 0.153 mmol) a été solubilisé dans un mélange d'eau (1 ml) et de THF (2 ml) à température ambiante et agité 16 h à cette température, puis co-évaporé 4 fois à sec à 80°C avec un mélange THF-eau et chromatographié sur C18. Le composé **104** pur a été obtenu (28 mg, rendement=23%). La molécule **104** a été caractérisée par son spectre de masse et RMN 1H et 31P.

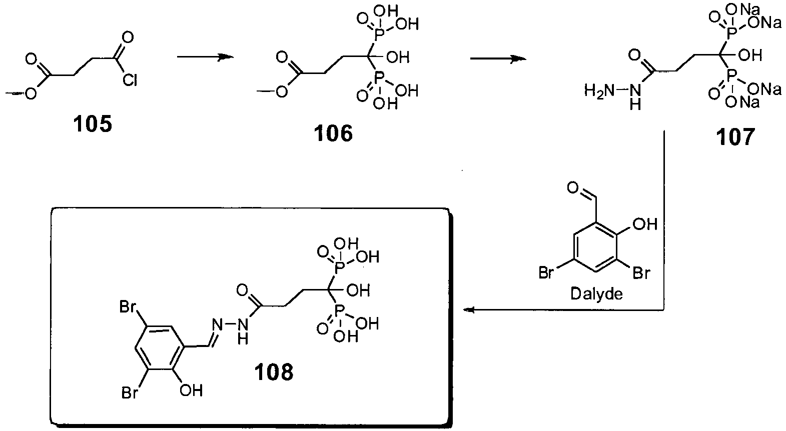

### Composé 107 :

Le mélange de **105** (166 µl, 1.34 mmol) et de tris(triméthylsilyl)phosphite (800 mg, 2.68 mmol) a été agité pendant 30 min sous Ar, puis le MeOH (2 ml) a été rajouté et le mélange a été agité 15 min puis concentré à sec à 60°C. Le produit brut obtenu a été dissous dans NH₂NH₂·H₂O et agité 3 h à TA. 2 éq de NaOH ont été rajoutés suivi de MeOH et le précipité formé a été filtré, redissous dans 2 ml d'eau et cristallisé de MeOH puis filtré. Le produit obtenu a été solubilisé dans l'eau (3 ml), 3 éq de NaOH y ont été rajoutés puis la solution a été lyophilisée. Ainsi le **107** pur a été obtenu, 230 mg, rendement=93%. La molécule **107** a été caractérisée par son spectre de masse et RMN 1H et 31P.

### Composé 108 :

Il a été préparé de la même façon que le composé **73** en partant de **107** et de Dalyde et a été caractérisé.
- La molécule **111** de référence, portant un résidu d'anesthésique local benzocaïne, a été synthétisée selon le schéma réactionnel suivant :

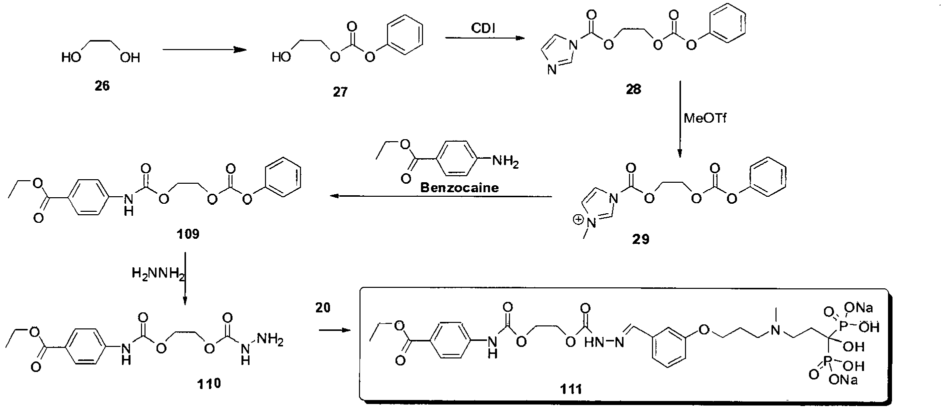

### Composé 109 :

Il a été préparé de la même façon que le composé **30** en partant du composé **28** et de benzocaïne et a été caractérisé.

### Composé 110 :

Il a été préparé de la même façon que le composé **31** en partant du composé **109** et a été caractérisé.

### Composé 111 :

Il a été préparé de même façon que le composé **32** en partant des composés **110** et **20** et a été caractérisé par son spectre de masse et RMN 1H et 31P.
- L'analogue fluorescent de **3** - la molécule **115** de référence - portant un résidu de dansyle a été synthétisé selon le schéma réactionnel suivant :

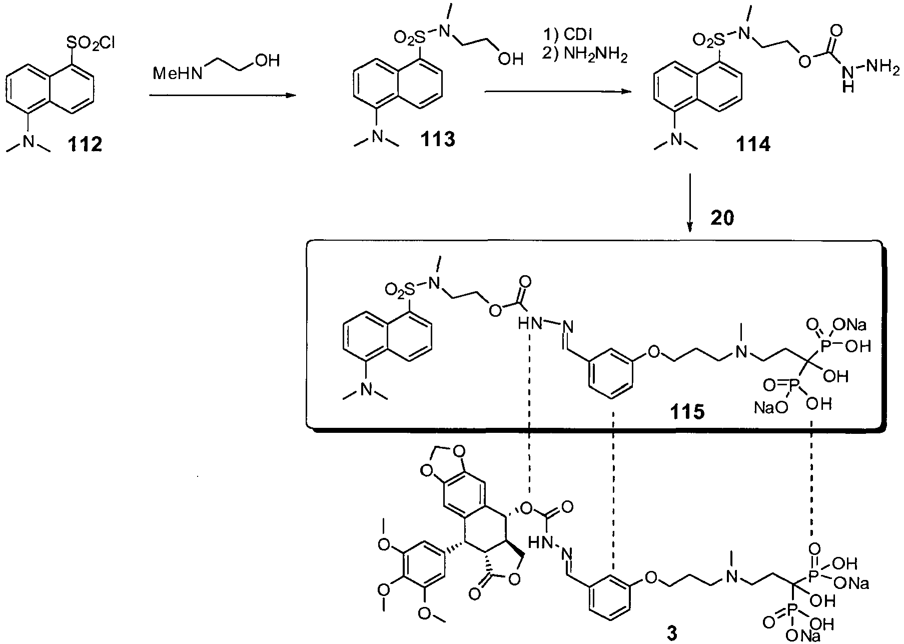

### Composé 114 :

Il a été préparé de la même façon que le composé **22** en partant du composé **113** et a été caractérisé.

### Composé 115 :

Il a été préparé de la même façon que le composé **3** en partant des composés **114** et **20** et a été caractérisé par son spectre de masse et RMN 1H et 31P.
- Des dérivés fluorescents - les molécules **124, 125, 126** et **127** de référence - portant un résidu de dansyle ont été synthétisés selon le schéma réactionnel suivant :

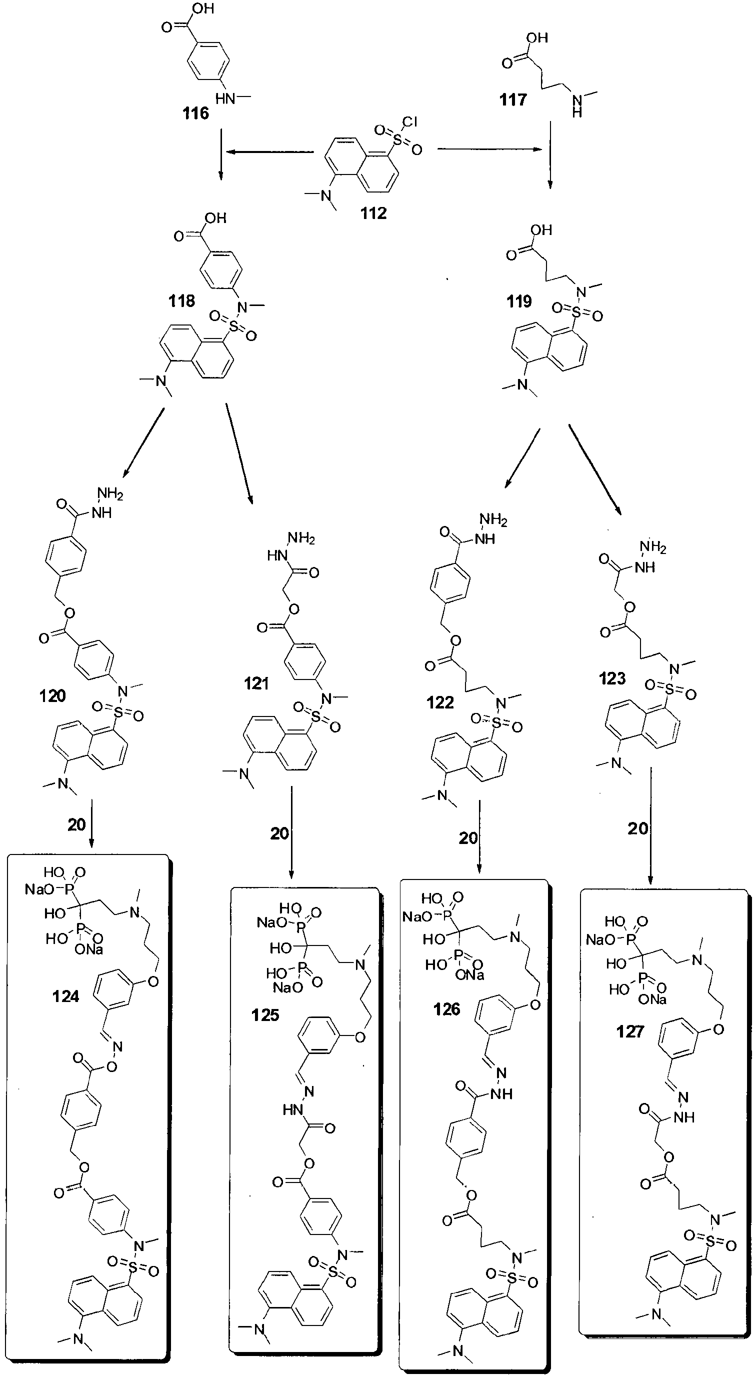

### Composés 120, 121, 122 et 123 :

Ils ont été préparés de la même façon que le composé **75** en partant respectivement des composés **118, 119**, **60** et **76** et ont été caractérisés.

### Composés 124, 125, 126 et 127 :

Ils ont été préparés de la même façon que les composés **73, 80** et **90** en partant respectivement des composés **120, 121, 122, 123** et **20** et ont été caractérisés par leurs spectres de masse et RMN 1H et 31P.
- Un dérivé fluorescent - la molécule **131** de référence - portant un résidu de dansyle a été synthétisé selon le schéma réactionnel suivant :

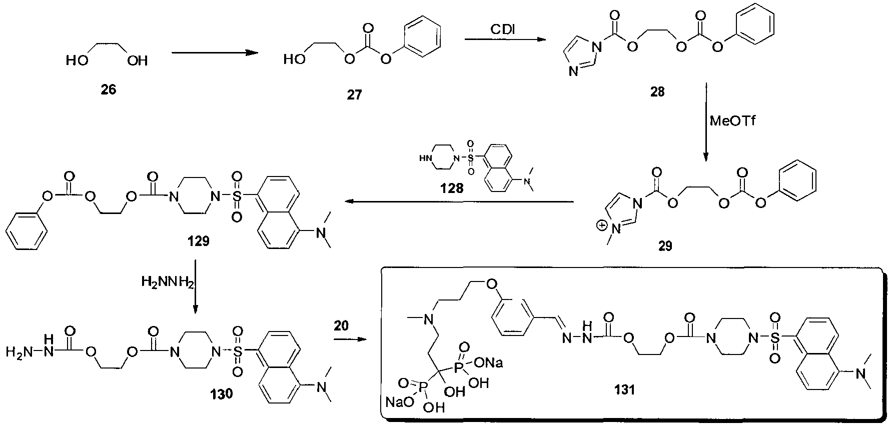

### Composé 129 :

Il a été préparé de la même façon que le composé **30** en partant des composés **28** et **128** commerciaux et a été caractérisé.

### Composé 130 :

Il a été préparé de la même façon que le composé **31** en partant du composé **129** et a été caractérisé.

### Composé 131 :

Il a été préparé de la même façon que le composé **32** en partant des composés **130** et **20** et a été caractérisé par son spectre de masse et RMN 1H et 31P.

### 2. Test de dissociation des composés dans des milieux biologiques

La dissociation de la liaison imine X₂ et la libération des composés d'intérêt biologiques ont été démontrées. Dans ce but, les dérivés fluorescents ont été utilisés pour une meilleure détection des produits de dissociation dans des milieux biologiques comme le broyat de tumeur, le sérum physiologique, etc.

Ce test de dissociation a notamment été réalisé avec la molécule **115** de référence portant un résidu de dansyle qui représente notamment un analogue de la molécule **3** selon l'invention, le motif podophyllotoxine ayant été remplacé par un groupe dansyle. Pour cela, le composé **115** est placé en milieu broyat de tumeur à 37°C et des mesures par HPLC sont effectuées afin d'observer la cinétique de libération du composé 114 et du composé 113 (voir schéma ci-dessous).

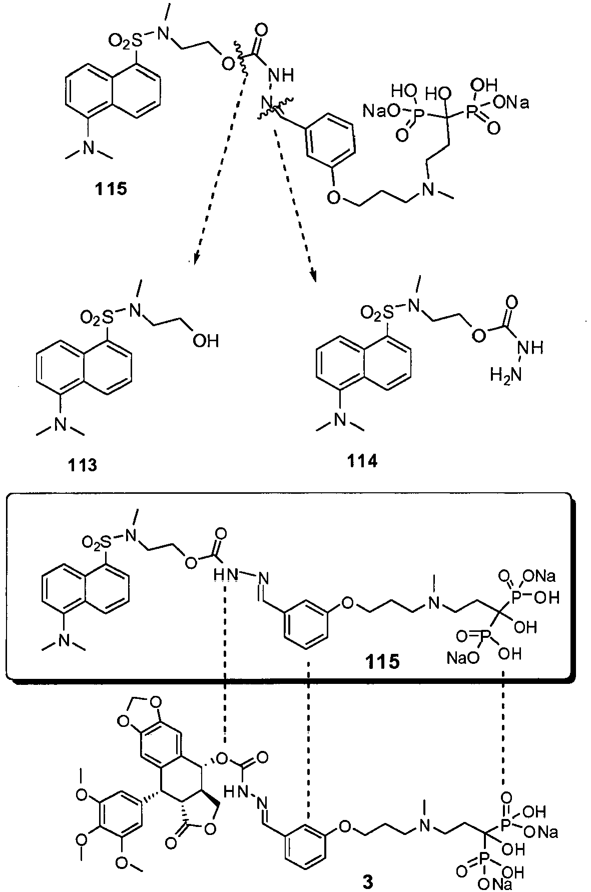

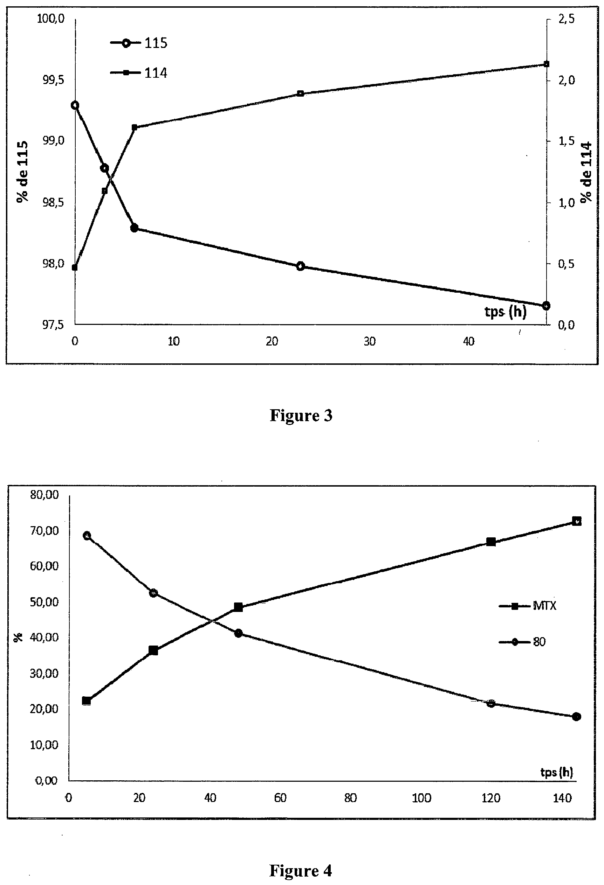

Les résultats obtenus sont présentés sur la figure 3.

Ce test de dissociation a également été effectué avec la molécule 80 selon l'invention. Pour cela, le composé **80** selon l'invention est placé en milieu broyat de tumeur à 37°C et des mesures par HPLC sont effectuées afin d'observer la cinétique de la libération du méthotréxate (MTX).

Les résultats obtenus sont présentés sur la figure 4.

### 3. Tests biologiques

Les résultats des tests de dissociation des composés selon l'invention dans des milieux biologiques, où la dissociation de la liaison imine et la libération des composés d'intérêt biologiques ont été démontrées, sont en accord avec l'activité biologique de ces composés selon l'invention.

### • Etude in vitro

Des études ont été menées pour évaluer le potentiel cytotoxique ou anti-prolifératif *in vitro* des composés selon l'invention sur trois lignées cellulaires : deux lignées tumorales (POS1, cellules d'ostéosarcome de souris ; SaOS2, cellules d'ostéosarcome humain) et une lignée non tumorale fibroblastique (L929, fibroblastes d'origine murine).

Les cellules d'ostéosarcome de souris (POS1) ont été cultivées dans un milieu RPMI (Roswell Park Memorial Institute), SVF (Sérum de Veau Foetal) (5%), dans une atmosphère humide à 37°C contenant 5 % de CO₂.

Les cellules d'ostéosarcome humain (SaOS2) ont été cultivées dans un milieu DMEM (Dulbecco's Modified Eagle Medium), SVF (Sérum de Veau Foetal) (10%), dans une atmosphère humide à 37°C contenant 5 % de CO₂.

Les fibroblastes d'origine murine (L929) ont été cultivés dans un milieu RPMI, SVF (5%), dans une atmosphère humide à 37°C contenant 5% de CO₂.

Pour réaliser les tests de cytotoxicité, les différentes lignées cellulaires sont ensemencées (1000 cellules par puits) à To dans des plaques 96 puits.

Les puits périphériques sont complétés avec 100 µL de milieu de culture seul. Les concentrations du composé à tester sont mises à contact à T₂₄ (24 heures après l'ensemencement).

Un test XTT (kit XTT, fournisseur ROCHE) est réalisé après 72 h de mise en contact des cellules avec les différents extraits aux différentes concentrations. Une lecture à 4 heures est réalisée sur un lecteur de plaques VICTOR 2, 1420 multilabel counter, PERKIN ELMER.

Ce test permet de mesurer l'activité de la déshydrogénase mitochondriale, enzyme présente uniquement dans les cellules vivantes. Cette enzyme clive le XTT libérant des cristaux de formazan oranges solubles dans le milieu de culture. L'intensité de coloration est mesurée par spectrophotométrie.

Des tests de cytotoxicité ont été réalisés pour les composés **3, 41, 42, 46** et **80** selon l'invention solubilisés dans 0,5% de tween 20, après 72 heures de culture en présence du composé à tester (plusieurs concentrations de 0,005 µM à 100 µM).

Chaque concentration a été évaluée en triplicat pour le test de viabilité cellulaire. Chaque gamme de concentrations testées a été préparée extemporanément à partir du composé.

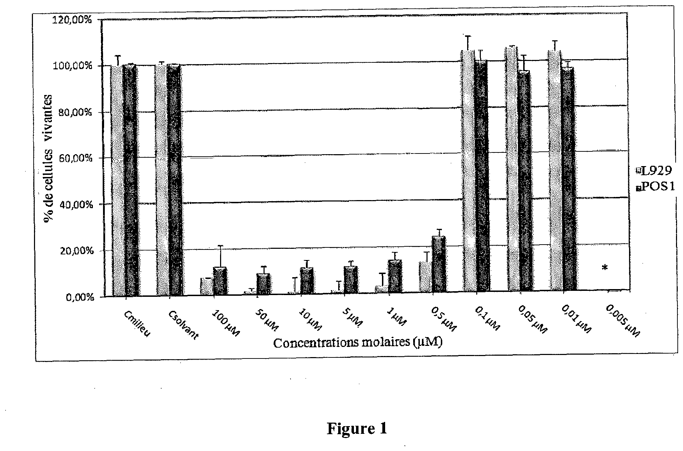

Les résultats obtenus sont présentés sur la figure 1 pour le composé **3** selon l'invention. Le test de viabilité cellulaire pratiqué après 72 heures de contact entre les différentes lignées cellulaires et le composé **3** met en évidence un effet anti prolifératif ou cytotoxique sur les deux lignées cellulaires aux concentrations de 0,5 µM à 100 µM pour la lignée tumorale POS1 et la lignée non tumorale L929.

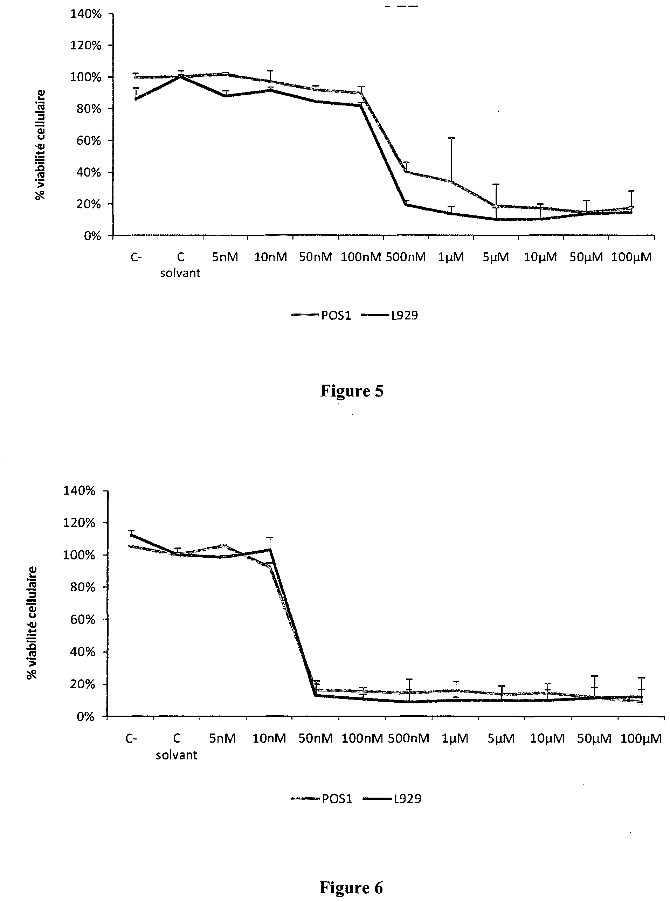

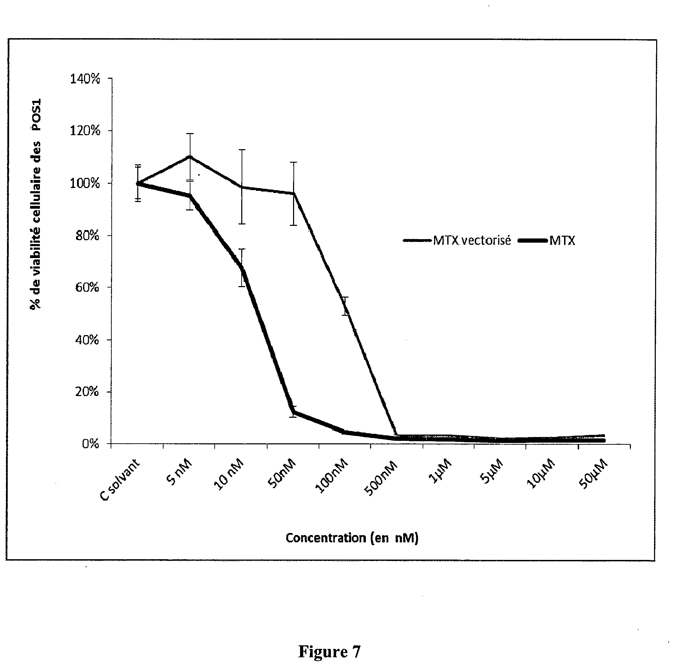

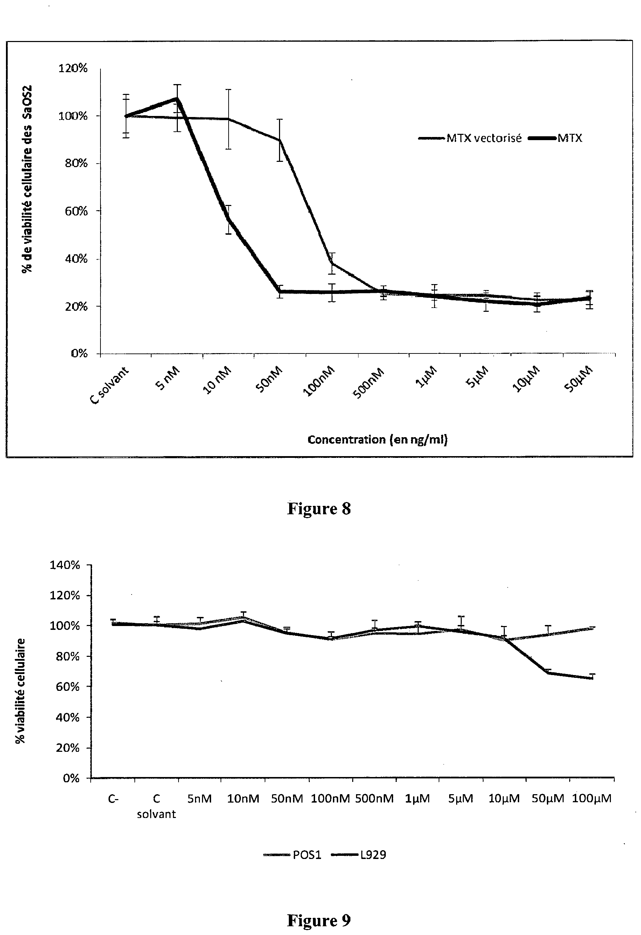

Les résultats obtenus sur POS1 et L929 sont présentés sur la figure 5 pour le composé 3 selon l'invention (podophyllotoxine vectorisée) et sur la figure 6 pour le composé 21 (podophyllotoxine). Les résultats obtenus avec le composé 80 selon l'invention (méthotréxate vectorisé) et le méthotréxate (MTX) sont présentés sur la figure 7 pour POS1 et sur la figure 8 pour SaOS2.

Pour la lignée **POS1,** des diminutions de la viabilité cellulaire compris entre 75% et 90% comparativement au contrôle sont enregistrées aux concentrations de 0,5 µM à 100 µM.

Pour la lignée **L929,** des diminutions de la viabilité cellulaire compris entre 87% et 99% comparativement au contrôle sont enregistrées aux concentrations de 0,5 µM à 100 µM. Il a été constaté en outre que les composés **41, 42** et **46** selon l'invention induisaient une diminution de la viabilité cellulaire associée à un effet-dose aux concentrations 5 µM, 10 µM, 50 µM et 100 µM pour la lignée tumorale POS1, seule lignée testée.

Les CI50 sont comprises entre 1 µM et 5 µM pour tous les composés testés.

Il a ainsi été démontré que les composés selon l'invention possédant une liaison imine et un groupe R1 représentant un résidu de composé anticancéreux possèdent une activité biologique anticancéreuse ce qui n'est pas le cas de la molécule (III) de la demande de brevet WO 2009/083614 où un autre type d'accroche que la liaison imine est utilisé, comme montré sur la figure 9.

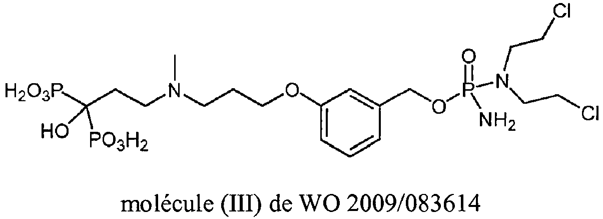

### • Etude in vivo

### - Effet antitumoral du composé 3 selon l'invention

La molécule **3** a été testée *in vivo* dans un modèle d'ostéosarcome où une transplantation de cellules tumorales murines (POS-1) a été effectuée à proximité des tibias de souris selon un protocole décrit dans Ory B. et al. Cancer 2005, 104(11), 2522-2529. La tumeur se développe environ 14 jours après la transplantation et progresse rapidement. Elle peut être à l'origine de métastases pulmonaires augmentant la mortalité des animaux. La molécule **3** à la concentration de 0,5 µmol/kg réduit la progression tumorale lorsque le composé est administré après l'établissement des tumeurs *in vivo.*

### - Utilisation du composé 50 de référence pour l'imagerie médicale par fluorescence

La molécule fluorescente **50** a été testée pour l'imagerie médicale par fluorescence *in vivo* sur des souris Swiss mâles, âgées de 6 semaines. Le principe fluorescent non vectorisé **49** a été testé dans les mêmes conditions, à savoir 50 µmol/kg injecté (sous forme d'une solution dans une solution aqueuse de NaCl 0,9%) par voie intra-péritonéale, avec un temps de diffusion de 120 min après l'injection des molécules.

Des souris ont reçu également une injection de véhicule seul (solution aqueuse de NaCl 0,9%) pour servir de contrôle négatif.

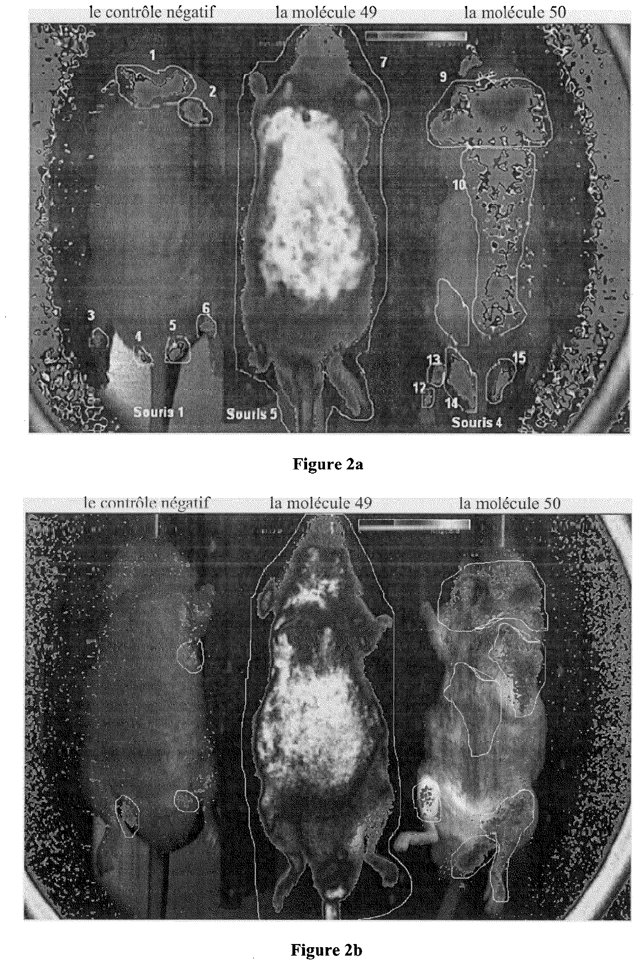

Les résultats du test sont présentés sur les figures 2a et 2b. Il apparaît clairement que la répartition de fluorescence pour les molécules **49** et **50** est différente, la molécule vectorisée **50** donnant une localisation de fluorescence moins diffuse, située plutôt au niveau des os.

## Revendications

1. Dérivé d'acide hydroxy-bisphosphonique bifonctionnel choisi parmi : 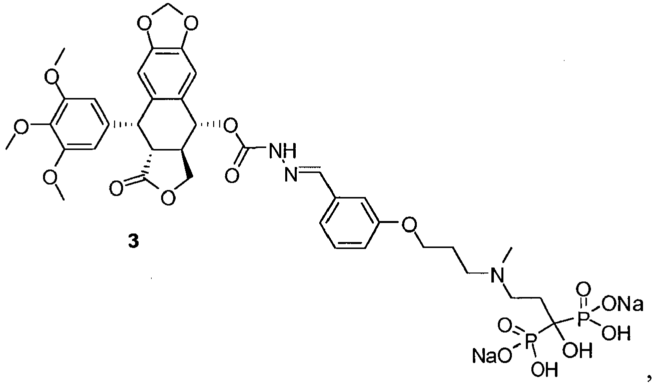 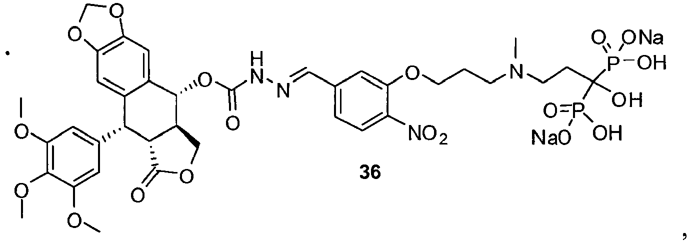 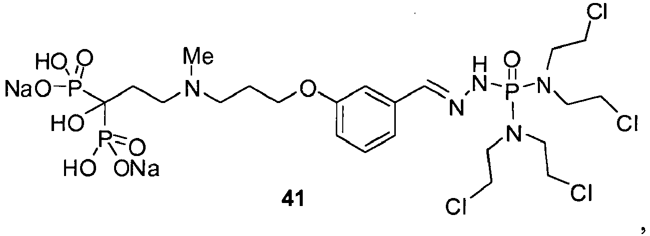 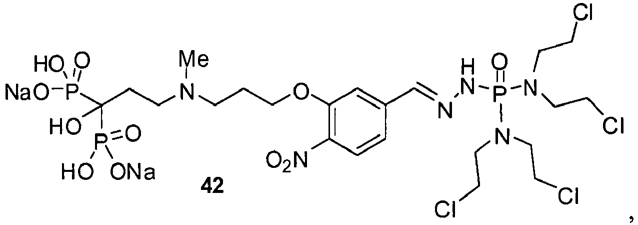 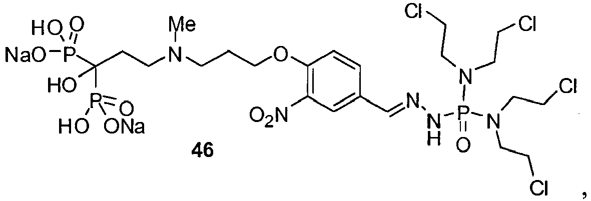 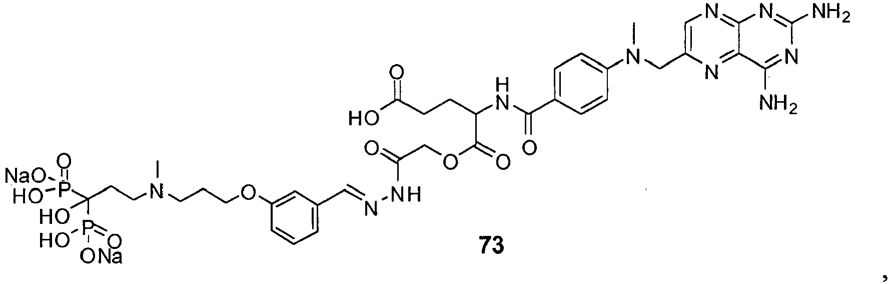 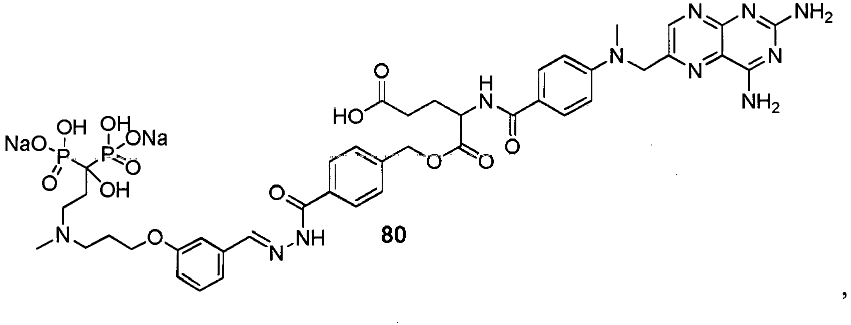 et 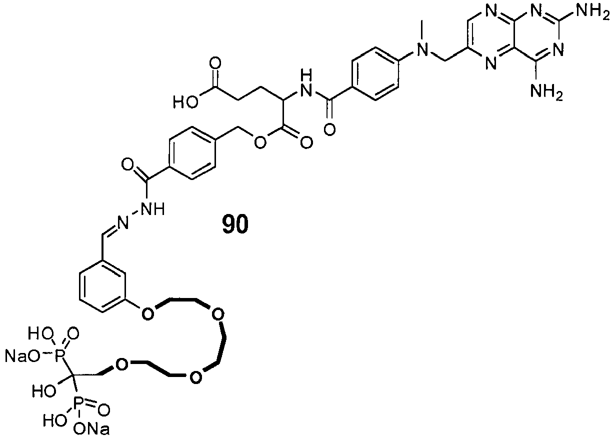 ou un sel pharmaceutiquement acceptable de celui-ci.

2. Dérivé d'acide hydroxy-bisphosphonique bifonctionnel selon la revendication 1 pour son utilisation en tant que médicament, notamment pour cibler le tissu osseux.

3. Dérivé d'acide hydroxy-bisphosphonique bifonctionnel selon la revendication 2, **caractérisé en ce que** le médicament est destiné au traitement d'une pathologie du remodelage osseux ostéolytique ou ostéocondensante choisie parmi les tumeurs osseuses primitives comme un ostéosarcome, un chondrosarcome, une tumeur à cellules géantes ou un sarcome d'Ewing ; les métastases osseuses et le myélome multiple.

4. Composition pharmaceutique comprenant au moins un dérivé d'acide hydroxy-bisphosphonique bifonctionnel selon la revendication 1 et au moins un véhicule pharmaceutiquement acceptable.

5. Composé choisi parmi : 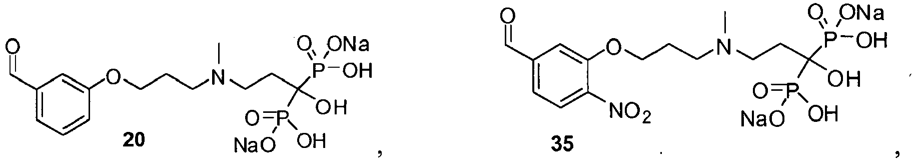 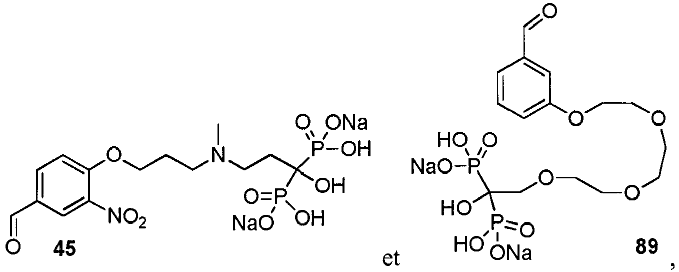 ou un sel pharmaceutiquement acceptable de celui-ci.

## Patentansprüche

1. Bifunktionelles Hydroxybisphosphonsäurederivat, ausgewählt unter : 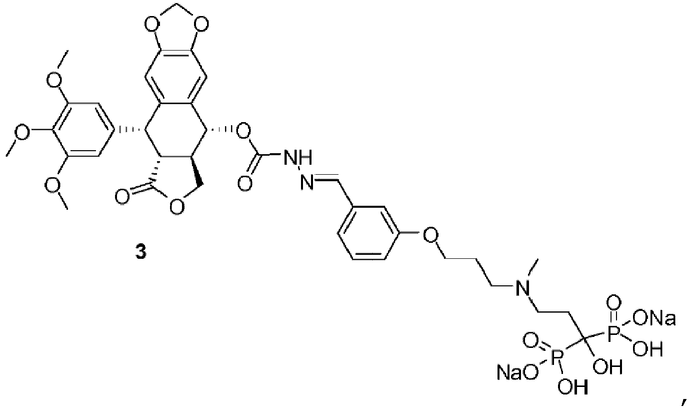 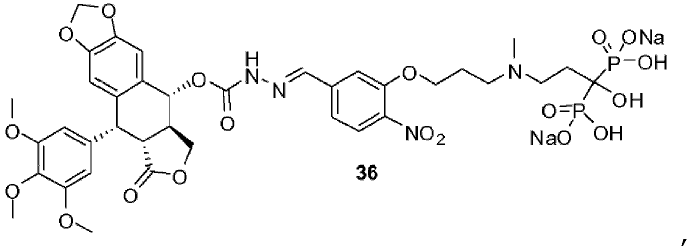 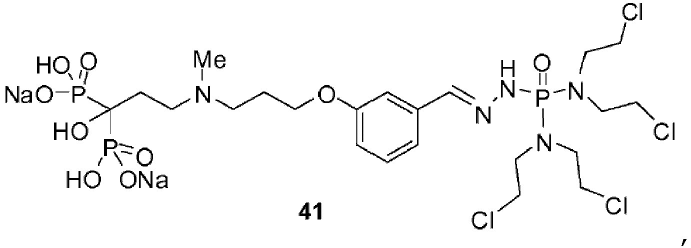 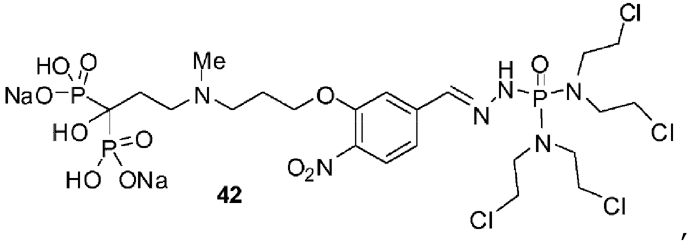 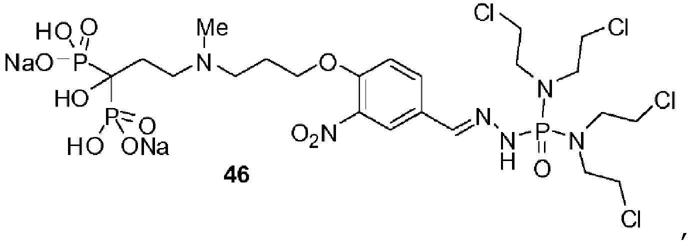 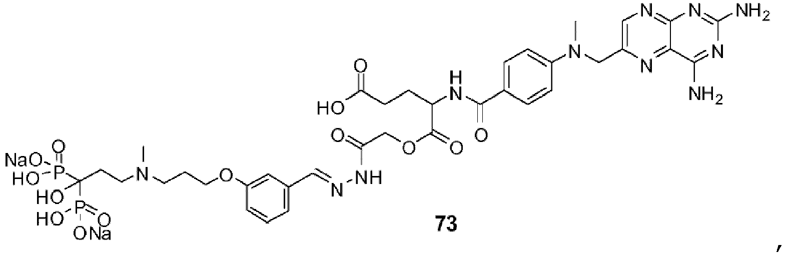 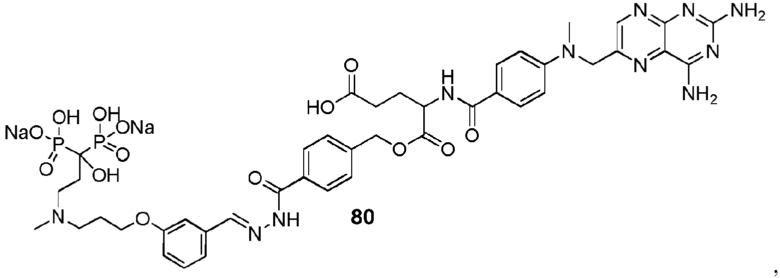 und 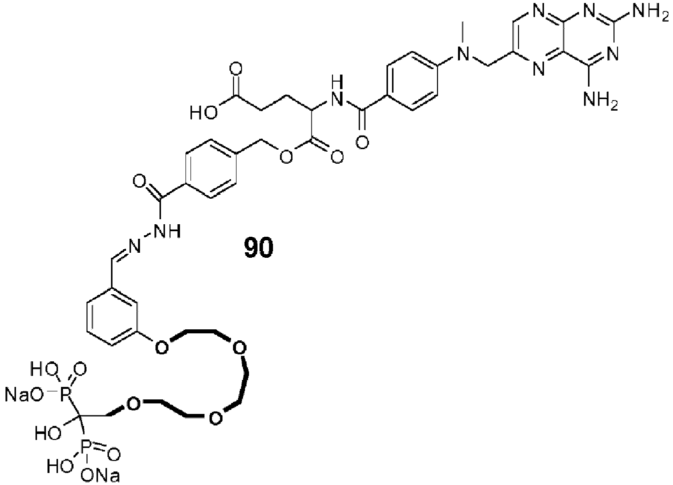 oder ein pharmazeutisch akzeptables Salz davon.

2. Bifunktionelles Hydroxybisphosphonsäurederivat nach Anspruch 1 zur Verwendung als Medikament, insbesondere zur Bekämpfung von Knochengewebe.

3. Bifunktionelles Hydroxybisphosphonsäurederivat nach Anspruch 2, **dadurch gekennzeichnet, dass** das Medikament zur Behandlung einer osteolytischen oder osteokondensierenden Knochenumgestaltungspathologie bestimmt ist, die unter primären Knochentumoren wie Osteosarkom, Chondrosarkom, Riesenzelltumor oder Ewing-Sarkom; Knochenmetastasen und multiplem Myelom ausgewählt ist.

4. Pharmazeutische Zusammensetzung, umfassend zumindest ein bifunktionelles Hydroxybisphosphonsäurederivat nach Anspruch 1 und zumindest einen pharmazeutisch verträglichen Träger.

5. Verbindung, ausgewählt unter: 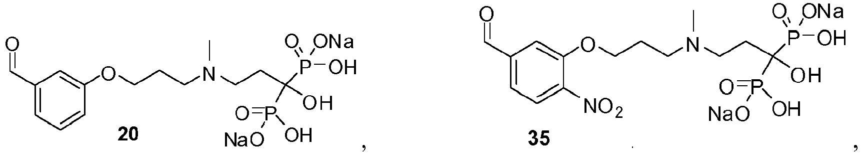 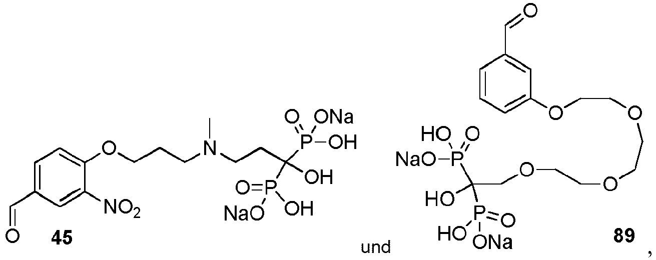 oder ein pharmazeutisch akzeptables Salz davon.

## Claims

1. A bifunctional hydroxy-bisphosphonic acid derivative selected from: 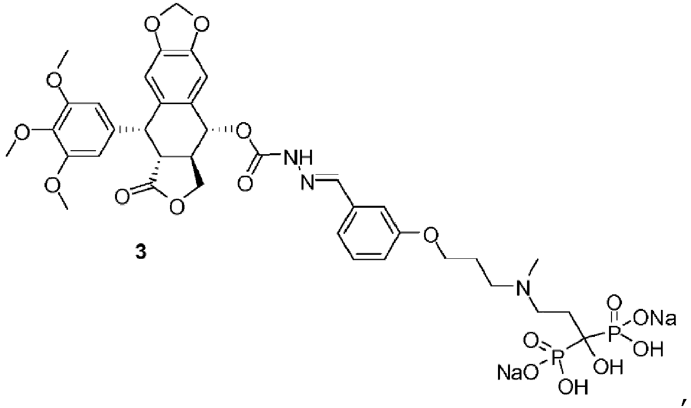 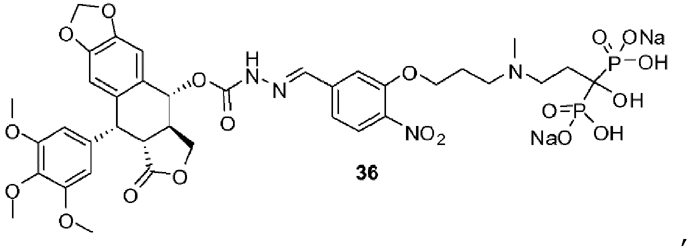 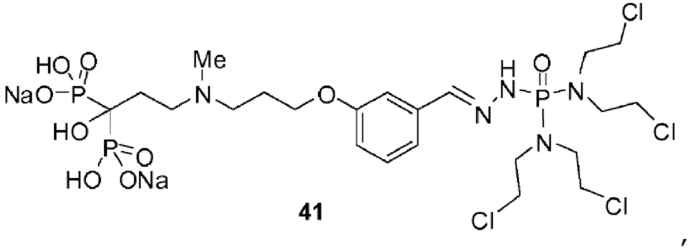 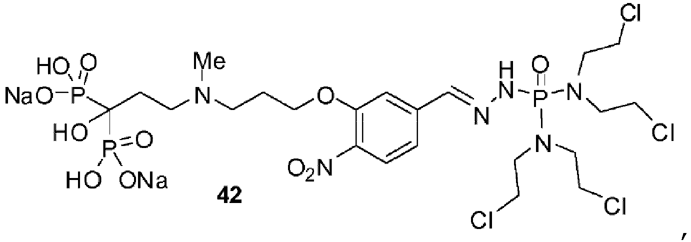 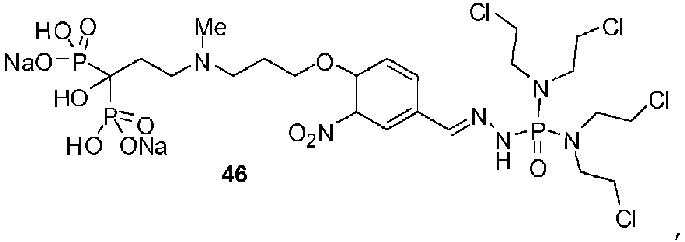 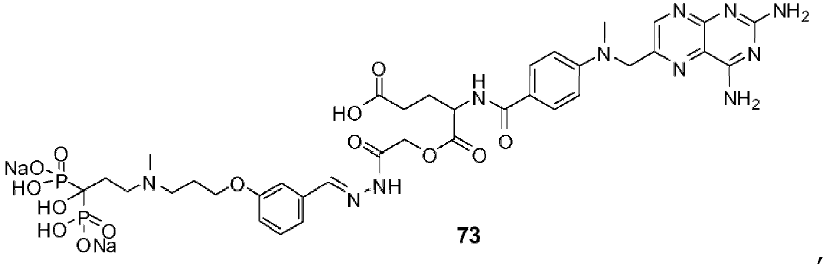 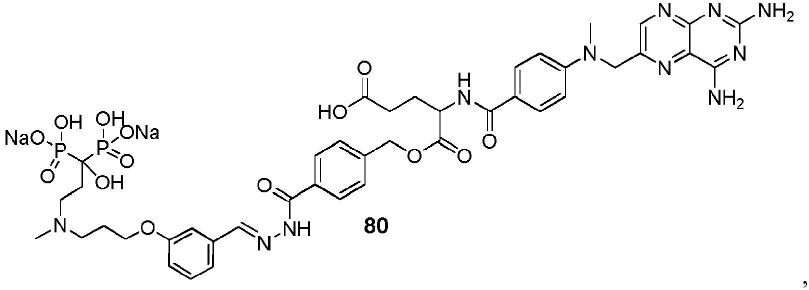 and 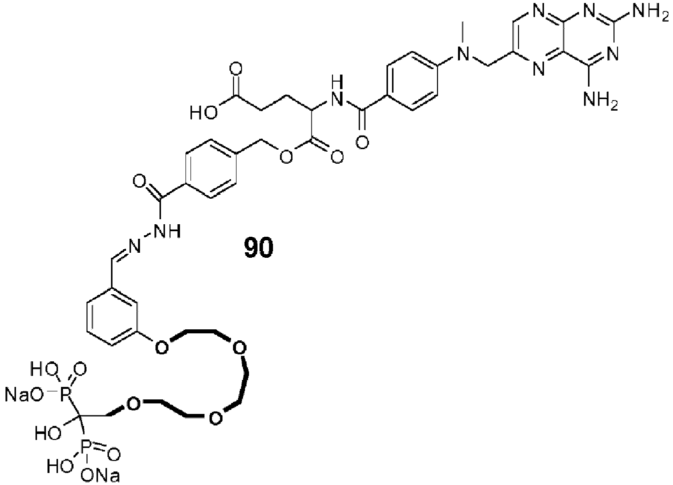 or a pharmaceutically acceptable salt thereof.

2. The bifunctional hydroxy-bisphosphonic acid derivative according to claim 1 for use as a medicinal product, especially for targeting bone tissue.

3. The bifunctional hydroxy-bisphosphonic acid derivative according to claim 2, **characterised in that** the medicinal product is intended for the treatment of an osteolytic or osteocondensing bone remodelling disease selected from primary bone tumours like osteosarcoma, chondrosarcoma, a giant cell tumour or Ewing's sarcoma; bone metastases and multiple myeloma.

4. A pharmaceutical composition comprising at least one bifunctional hydroxy-bisphosphonic acid derivative according to claim 1 and at least one pharmaceutically acceptable carrier.

5. A compound selected from: 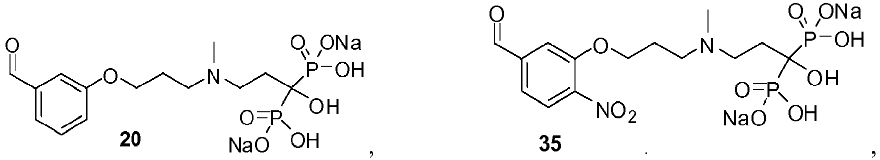 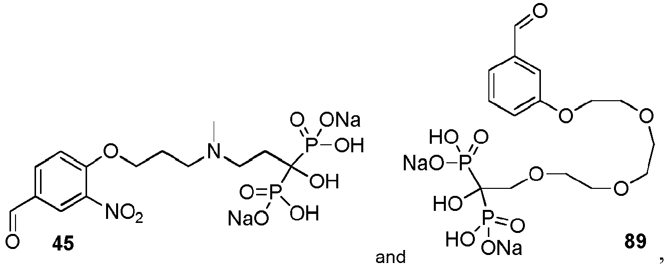 or a pharmaceutically acceptable salt thereof.
